# EUROPÄISCHE PATENTSCHRIFT

(11) **EP 0 669 315 B1**
(45) Veröffentlichungstag und Bekanntmachung des Hinweises auf die Patenterteilung: **06.08.1997**
(21) Anmeldenummer: 95106286.8
(22) Anmeldetag: 07.09.1991
(51) Int. Cl.: C07C 251/48, C07C 255/64, A01N 37/50

(54) **Ortho-substituierte Phenylessigsäureamide**
Ortho substituted phenylacetic acid amides
Amides d'acides phénylacétiques ortho-substitués

(30) Priorität: 22.09.1990 DE 4030038
(43) Veröffentlichungstag der Anmeldung: 30.08.1995
(62) Teilanmeldung aus: 91115145.4
(73) Patentinhaber: BASF AKTIENGESELLSCHAFT, 67056 Ludwigshafen (DE)
(72) Erfinder: Brand, Siegbert, Dr., D-67346 Speyer (DE); Ammermann, Eberhard, Dr., D-64646 Heppenheim (DE); Lorenz, Gisela, Dr., D-67434 Hambach (DE); Sauter, Hubert, Dr., D-68167 Mannheim (DE); Oberdorf, Klaus, Dr., D-69117 Heidelberg (DE); Kardorff, Uwe, Dr., D-68159 Mannheim (DE); Künast, Christoph, Dr., D-67166 Otterstadt (DE)

(56) Entgegenhaltungen:
- EP-A- 0 088 325
- EP-A- 0 398 692
- CH-A- 636 601
- DE-A- 2 808 317

## Beschreibung

Die vorliegende Erfindung betrifft ein Verfahren zur Bekämpfung von Schädlingen, welches dadurch gekennzeichnet ist, daß man eine insektizid, nematizid und/oder akarizid wirksame Menge eines ortho-substituierten Phenylessigsäureamides der allgemeinen Formel I 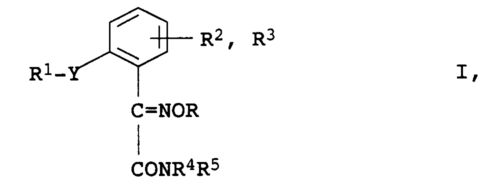 in der die Variablen die folgende Bedeutung haben:
- R: eine C₁-C₄-Alkylgruppe,
- R¹: Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe oder
eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anneliert sein kann und wobei der Heterocyclus zusätzlich noch ein Halogenatom, einen oder zwei C₁-C₄-Alkylreste oder einen Phenylrest tragen kann;
- R², R³: unabhängig voneinander Wasserstoff, Cyano, Halogen, C₁-C₄-Alkyl oder C₁-C₄-Alkoxy;
- R⁴, R⁵: unabhängig voneinander Wasserstoff oder Methyl;
- Y: Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette,
ausgenommen solche Verbindungen, in denen die Gruppe NR⁴R⁵ für NHCH₃ steht, Y Sauerstoff bedeutet, R² und R³ für Wasserstoff stehen, R für Methyl steht und R¹ Alkenyl bedeutet oder Phenyl bedeutet, welches
- einen der folgenden Reste trägt: Nitro, Halogen, Methyl, Isopropyl, tert.-Butyl, Methoxy, Phenoxy oder unsubstituiertes oder substituiertes Benzyloxy, oder welches
- zwei Chloratome oder zwei Isopropylgruppen trägt;
ausgenommen solche Verbindungen, in denen R² und R³ für Wasserstoff stehen, die Gruppe NR⁴R⁵ für NHCH₃ steht, Y OCH₂ bedeutet, R für Methyl steht und R¹ Phenyl bedeutet, welches ein Chloratom trägt; sowie
ausgenommen solche Verbindungen, in denen R² und R³ für Wasserstoff stehen, die Gruppe NR⁴R⁵ für NHCH₃ steht, Y OCH₂ bedeutet und R¹ für eine der folgenden Gruppen steht: 3-Phenoxyphenyl, 4-Cyanophenyl, 3-Nitrophenyl, 3-(N,N-Dimethylamino)-phenyl, 4-Fluorphenyl, 3-Bromphenyl, 4-Bromphenyl, 2,4-Dichlorphenyl, 3,4-Dichlorphenyl, 3,5-Dichlorphenyl, 4-Methylphenyl, 4-tert.-Butylphenyl, 3-Trifluormethylphenyl, 4-Methoxyphenyl, 3-Isopropyloxvphenyl, Pyrimidin-2-yl oder Pyridin-2-yl,
auf Insekten, Nematoden und/oder Akaridien bzw. deren Lebensraum einwirken läßt.

Des weiteren betrifft die Erfindung neue ortho-substituierte Phenylessigsäureamide der allgemeinen Formel I, 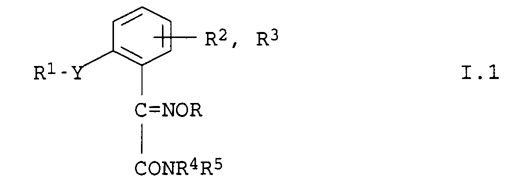 wobei die Variablen folgende Bedeutung haben:
- R: eine C₁-C₄-Alkylgruppe,
- R¹: Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe oder
eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anneliert sein kann und wobei der Heterocyclus zusätzlich noch ein Halogenatom, einen oder zwei C₁-C₄-Alkylreste oder einen Phenylrest tragen kann;
- R², R³: unabhängig voneinander Wasserstoff, Cyano, Halogen, C₁-C₄-Alkyl oder C₁-C₄-Akoxy;
- R⁴,: Wasserstoff oder eine C₁-C₄-Alkylgruppe ;
- R⁵: eine C₁-C₄-Alkoxygruppe;
- Y: Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette.

Daneben betrifft die Erfindung neue ortho-substituierte Phenylessigsäureamide der allgemeinen Formel I.2 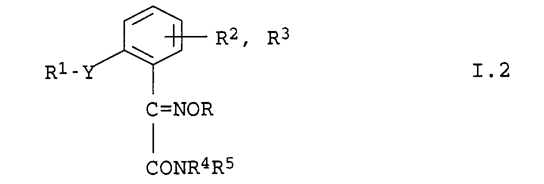 wobei die Variablen folgende Bedeutung haben:
- R: eine C₁-C₄-Alkylgruppe,
- R¹: Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe oder
eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl,
C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anneliert sein kann und wobei der Heterocyclus zusätzlich noch ein Halogenatom, einen oder zwei C₁-C₄-Alkylreste oder einen Phenylrest tragen kann;
- R²: Wasserstoff, Cyano, Halogen, C₁-C₄-Alkyl oder C₁-C₄-Akoxy;
- R³: Cyano;
- R⁴, R⁵: unabhängig voneinander Wasserstoff oder eine C₁-C₄-Alkylgruppe;
- Y: Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette.

Gleichermaßen betrifft die Erfindung neue ortho-substituierte Phenylessigsäureamide der allgemeinen Formel I.3 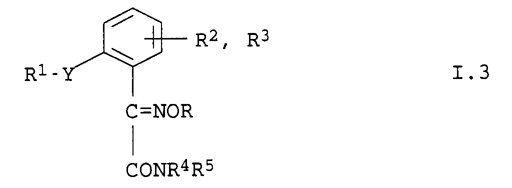 wobei die Variablen folgende Bedeutung haben:
- R: eine C₁-C₄-Alkylgruppe,
- R¹: Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe oder
eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl,
C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anneliert sein kann und wobei der Heterocyclus zusätzlich noch ein Halogenatom, einen oder zwei C₁-C₄-Alkylreste oder einen Phenylrest tragen kann;
- R², R³: unabhängig voneinander Wasserstoff, Cyano, Halogen, C₁-C₄-Alkyl oder C₁-C₄-Akoxy;
- R⁴, R⁵: unabhängig voneinander Wasserstoff oder eine C₁-C₄-Alkylgruppe oder der beiden Substituenten eine C₁-C₄-Alkoxygruppe;
- Y: eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine Carbonyl-C₁-C₄-alkenylen- oder C₁-C₄-Alkylencarbonylkette.

Außerdem betrifft die Erfindung neue ortho-substituierte Phenylessigsäureamide der allgemeinen Formel I.4 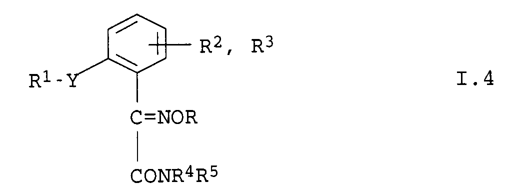 wobei die Variablen folgende Bedeutung haben:
- R: eine C₁-C₄-Alkylgruppe,
- R¹: Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₁-C₄-Alkoxy-C₁-C₄-alkylgruppe, eine C₁-C₄-Alkoxycarbonylgruppe,
- R², R³: unabhängig voneinander Wasserstoff, Cyano, Halogen, C₁-C₄-Alkyl oder C₁-C₄-Akoxy;
- R⁴, R⁵: unabhängig voneinander Wasserstoff oder eine C₁-C₄-Alkylgruppe oder eine der beiden Substituenten eine C₁-C₄-Alkoxygruppe;
- Y: Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette.

Daneben betrifft die Erfindung neue ortho-substituierte Phenylessigsäureamide der allgemeinen Formel I.5 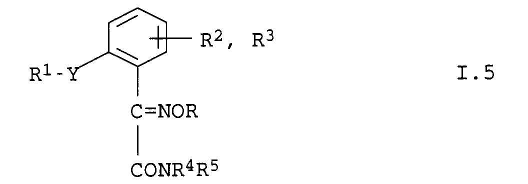 wobei die Variablen folgende Bedeutung haben:
- R: eine C₁-C₄-Alkylgruppe,
- R¹: Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe oder
eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus ausgewähtl aus der Gruppe: Pyrrol-2-yl, Pyrrol-3-yl, Furan-2-yl, Furan-3-yl, Thien-2-yl, Thien-3-yl, Pyrazol-3-yl, Pyrazol-4-yl, Pyrazol-5-yl, Oxazol-2-yl, Oxazol-4-yl, Oxazol-5-yl, Benzothiazol-2-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, 1,2,4-Oxadiazol-3-yl, 1,2,4-Oxadiazol-5-yl, 1,3,4-Oxadiazol-2-yl, 1,3,4-Thiadiazol-2-yl, 1,2,4-Triazol-3-yl, 1,3,4-Triazol-2-yl, Pyridin-3-yl, Pyridin-4-yl, Pyridazin-3-yl, pyridazin-4-yl, Pyrimidin-4-yl, pyrimidin-5-yl, Pyrazin-2-yl, 1,3,5-Triazin-2-yl und 1,2,4-Triazin-3-yl, wobei der Heterocyclus zusätzlich noch ein Halogenatim, einen oder zwei C₁-C₄-Alkylreste oder einen Phenylrest tragen kann;
- R², R³: unabhängig voneinander Wasserstoff oder eine C₁-C₄-Alkyl oder C₁-C₄-Akoxy;
- R⁴, R⁵: unabhängig voneinander Wasserstoff oder eine C₁-C₄-Alkylgruppe oder einer der beiden Substituenten eine C₁-C₄-Alkoxygruppe;
- Y: Sauerstoff oder Schwefel,
eine C₂-Alkylen- oder C₂-C₄-Alkinylenkette, eine Oxy-C₁-C₄-alkylen-, Thio-C₁-C₄-alkylen- oder C₁-C₄-Alkylenoxykette.

Des weiteren betrifft die Erfindung neue ortho-substituierte Phenylessigsäureamide der allgemeinen Formel I.6 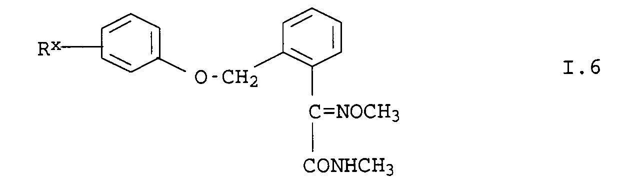 wobei R^{x} die folgenden Substituenten bzw. Substituentenkombinationen steht:
2-Fluor, 3-Fluor, 2-Brom, 2-Iod, 2-Methyl, 3-Methyl, 2-Methoxy, 3-Methoxy, 2-Trifluormethyl, 4-Trifluormethyl, 2-Nitro, 4-Nitro, 2-Chlormethyl, 3-Chlormethyl, 4-Chlormethyl, 2-Ethyl, 3-Ethyl, 4-Ethyl, 3-iso-Propyl, 4-iso-Propyl, 3-tert.-Butyl, 3-Phenyl, 4-Phenyl, 3,5-Diethyl, 4-Cyclohexyl, 4-iso-Propyloxy, 4-tert.-Butyloxy, 4-Phenoxy, 3-Benzyloxy, 4-Benzyloxy, 2,3-Dichloro, 2,5-Dichloro, 2,6-Dichloro, 2,3,4-Trichloro, 2,3,5-Trichloro, 2,3,6-Trichloro, 2,3,5-Trichloro, Pnetachloro, Pentafluoro, 2-Fluor-4-chlor, 4-Fluor-2-chlor, 2-Methyl-4-tert.-butyl, 2-Methyl-4-cyclohexyl, 2-Methyl-4-propyl, 2,3-Diemethyl, 2,4-Diemthyl, 2,5-Dimethyl, 3,4-Dimethyl, 3,5-Dimethyl, 2,3,5-Trimethyl oder 2,4,6-Trimethyl.

Gleichermaßen betrifft die Erfindung neue ortho-substituierte Phenylessigsäureamide der allgemeinen Formel I.7 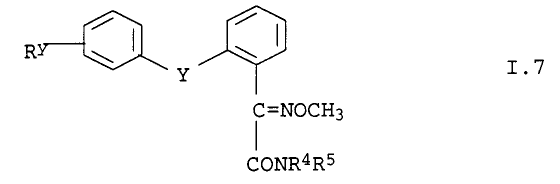 wobei die Variablen folgende Bedeutung haben:
- R⁴, R⁵: unabhängig voneinander Wasserstoff oder Methyl;
- Y: CH₂CH₂, CH=CH oder CH₂O,
- R^{y}: 2-Fluor, 3-Fluor, 4-Fluor, 2-Chlor, 3-Chlor, 4-Chlor, 2-Brom, 4-Brom, 2-Jod, 2-Methyl, 3-Methyl, 4-Methyl, 2-Methoxy, 3-Methoxy, 4-Methoxy, 2-Trifluormethyl, 3-Trifluormethyl, 4-Trifluormethyl, 2,4-Dichlor, 2,4-Dimethyl oder 2,4,6-Trimethyl.

Außerdem betrifft die Erfindung Verfahren zur Herstellung dieser Verbindungen I.1-I.7, ihre Verwendung als Fungizide sowie fungizide Mittel und Mittel zur Bekämpfung von Schädlingen, welche diese Verbindungen als wirksame Substanzen enthalten.

Ähnliche Verbindungen mit fungiziden Eigenschaften sind aus der nachveröffentlichten EP 398 692 bekannt.

Der Erfindung lagen neue fungizid wirksame ortho-substituierte Phenylessigsäurederivate sowie neue insektizide, akarizide und nematizide Wirkstoffe als Aufgabe zugrunde.

Demgemäß wurden die eingangs definierten ortho-substituierten Phenylessigsäureamide der Formel I (bzw. I.1 bis I.7) gefunden.

Im einzelnen haben die Substituenten in den erfindungsgemäßen Verbindungen I die folgende Bedeutung:
R¹
   - Wasserstoff;
   - eine verzweigte oder unverzweigte C₁-C₁₈-Alkylgruppe wie Methyl, Ethyl, n-Propyl, Isopropyl, 1,1-Dimethylprop-1-yl, 2,2-Dimethylprop-1-yl, n-Butyl, sec.-Butyl, Isobutyl, tert.-Butyl, 3-Methylbutyl, n-Pentyl, n-Hexyl, n-Heptyl, 2,6-Dimethylhept-1-yl, n-Octyl, n-Nonyl, n-Decyl, n-Pentadecyl, n-Heptadecyl und n-Octadecyl, bevorzugt eine C₁-C₁₀-Alkylgruppe;
   - eine C₃-C₈-Cycloalklgruppe wie Cyclopropyl, Cyclobutyl, Cyclopentyl, Cyclohexyl, Cycloheptyl und Cyclooctyl, die noch einen bis drei Substituenten tragen können, ausgewählt aus der einer Gruppe von 3 Halogenatomen wie Fluor, Chlor, Brom und Jod, insbesondere Fluor und Chlor, 3 C₁-C₄-Alkylgruppen wie Methyl, Ethyl, n-Propyl, Isopropyl, n-Butyl, sec.-Butyl und tert.-Butyl, einer partiell oder vollständig halogenierten C₁-C₄-Alkenylgruppe wie 2,2-Dichlorethenyl, und einer Phenylgruppe, die noch ein bis zwei Halogenatome wie vorstehend genannt, insbesondere Fluor und Chlor, und/oder eine C₁-C₄-Alkylgruppe wie Methyl, Ethyl, n-Propyl, Isopropyl, n-Butyl und tert.-Butyl und/oder eine C₁-C₄-Alkoxygruppe wie Methoxy, Ethoxy, n-Propoxy, Isopropoxy, n-Butoxy und tert.Butoxy tragen kann; bevorzugt sind Cyclopropyl, 1-Methylcycloprop-1-yl, 2,2-Dichlorcycloprop-1-yl, (2',2'-Dichlorvinyl)cycloprop-1-yl, 1-Phenylcycloprop-1-yl, 1-(p-Fluorphenyl)-cycloprop-1-yl, Cyclohexyl und 1-Methylcyclohex-1-yl;
   - eine C₂-C₁₀-Alkenylgruppe wie Vinyl, Allyl, Prop-1-en-1-yl, Prop-2-en-2-yl, 2-Methylprop-1-en-1-yl, But-2-en-1-yl, But-2-en-2-yl, 3-Methylbut-2-en-1-yl, 1,3-Pentadien-1-yl, 2,6-Dimethylhept-5-en-1-yl und 2, 6-Dimethyl-1,5-heptadien-1-yl;
   - eine C₂-C₄-Alkinylgruppe wie Ethinyl und Prop-2-in-1-yl, die noch einen Phenylrest tragen kann, z.B. 2-Phenylethinyl;
   - eine C₁-C₄-Alkoxy-C₁-C₄-Alkylgruppe wie Methoxymethyl, Ethoxymethyl, n-Propoxymethyl, Isopropoxymethyl, n-Butoxymethyl, tert.-Butoxymethyl, 1-Methoxyethyl, 2-Methoxyethyl, 1-Ethoxyethyl, 2-Ethoxyethyl und 2-n-Propoxyethyl);
   - eine C₁-C₄-Alkoxycarbonylgruppe wie Methoxycarbonyl, Ethoxycarbonyl, n-Propoxycarbonyl, Isopropoxycarbonyl, n-Butoxycarbonyl und tert.Butoxycarbonyl, bevorzugt Methoxycarbonyl;
   - eine Phenyl-C₁-C₄-alkylgruppe wie Benzyl, Phenethyl, 3-Phenyl-n-propyl und 4-Phenyl-n-butyl, eine Phenyl-C₂-C₄-alkenylgruppe wie Styryl und 2-Phenylprop-2-en-1-yl oder eine Phenoxy-C₁-C₄-alkylgruppe wie Phenoxymethyl, 2-Phenoxyethyl, 3-Phenoxypropyl und 4-Phenoxybutyl, wobei die genannten Gruppen am Phenylring jeweils noch insgesamt einen bis fünf Reste tragen können, davon insbesondere:
      - eine oder zwei Nitrogruppen,
      - eine oder zwei Cyanogruppen,
      - bis zu 5 Halogenatome wie vorstehend genannt, insbesondere Fluor und Chlor,
      - bis zu 3 C₁-C₄-Alkylgruppen wie vorstehend genannt,
      - bis zu 3 partiell oder vollständig halogenierte C₁-C₄-Alkylgruppen wie Fluormethyl, Chlormethyl, Trifluormethyl, Trichlormethyl, Dichlorfluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl,
      - bis zu 3 C₂-C₄-Alkenylgruppen wie Ethenyl, Prop-1-en-1-yl, Prop-2-en-1-yl, 1-Methylethen-1-yl, But-1-en-1-yl, But-2-en-1-yl, But-3-en-1-yl, 1-Methylprop-1-en-1-yl, 2-Methyl-prop-1-en-1-yl, 1-Methylprop-2-en-1-yl und 2-Methyl-prop-2-en-1-yl, insbesondere Ethenyl und Prop-2-en-1-yl,
      - bis zu 3 partiell oder vollständig halogenierte C₂-C₄-Alkenylgruppen wie 2-Fluorethenyl, 2-Chlorethenyl, Trifluorethenyl, Trichlorethenyl und 2-Chlorprop-2-en-1-yl und
      - bis zu 3 C₁-C₄-Alkoxygruppen wie Methoxy, Ethoxy, n-Propoxy, Isopropoxy, n-Butoxy und tert.-Butoxy,
      - eine Phenyl-, Benzyl-, Phenoxy-, Benzyloxy- oder Phenylthiogruppe, die ihrerseits noch einen oder zwei der folgenden Substituenten tragen kann: Cyano, Halogen wie vorstehend genannt, insbesondere Fluor und Chlor oder C₁-C₄-Alkyl wie vorstehend genannt;
      bevorzugt sind 2-Nitrophenyl, 4-Nitrophenyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Jodphenyl, 2,3-Dichlorphenyl, 2,4-Dichlorphenyl, 2,5-Dichlorphenyl, 2,6-Dichlorphenyl, 2,3,4-Trichlorphenyl, 2,3,5-Trichlorphenyl, 2,3,6-Trichlorphenyl, 3,4,5-Trichlorphenyl, Pentafluorphenyl, Pentachlorphenyl, 2-Methylphenyl, 3-Methylphenyl, 4-Methylphenyl, 2-Ethylphenyl, 3-Ethylphenyl, 4-Ethylphenyl, 3-Isopropylphenyl, 4-Isopropylphenyl, 3-tert.-Butylphenyl, 4-tert.-Butylphenyl, 2,3-Dimethylphenyl, 2,4-Dimethylphenyl, 2,5-Dimethylphenyl, 3,4-Dimethylphenyl, 3,5-Dimethylphenyl, 4-tert.-Butyl-2-methylphenyl, 3,5-Diethylphenyl, 2,3,5-Trimethylphenyl, 2,4,6-Trimethylphenyl, 4-Cyclohexylphenyl, 3-Phenoxyphenyl, 4-Phenoxyphenyl, 4-Phenylthiophenyl, 3-Benzyloxyphenyl, 4-Benzyloxyphenyl, 2-Trifluormethylphenyl, 3-Trifluormethylphenyl, 4-Trifluormethylphenyl, 2-Chlormethylphenyl, 3-Chlormethylphenyl, 4-Chlormethylphenyl, Benzyl, 4-Chlorbenzyl, Phenethyl, 4-Chlorphenethyl, Styryl, 4-Chlorstyryl, Phenoxy, 2-Chlorphenoxy, 3-Chlorphenoxy, 4-Chlorphenoxy, 2-Methylphenoxy, 3-Methylphenoxy, 4-Methylphenoxy, 2-Trifluormethylphenoxy, 3-Trifluormethylphenoxy, 4-Trifluormethylphenoxy, Phenoxymethyl und 2-Phenoxyethyl;
   - einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff- oder Schwefelatom anelliert sein kann, beispielsweise Pyrrol-2-yl, Pyrrol-3-yl, Furan-2-yl, Furan-3-yl, Thien-2-yl, Thien-3-yl, Pyrazol-3-yl, Pyrazol-4-yl, Pyrazol-5-yl, Oxazol-2-yl, Oxazol-4-yl, Oxazol-5-yl, Benzoxazol-2-yl, Thiazol-2-yl, Thiazol-4-yl, Thiazol-5-yl, Benzothiazol-2-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-3-yl, 1,3,4-Triazol-2-yl, Pyridin-2-yl, Pyridin-3-yl, Pyridin-4-yl, Pyridazin-3-yl, Pyridazin-4-yl, Pyrimidin-2-yl, Pyrimidin-4-yl, Pyrimidin-5-yl, Pyrazin-2-yl, 1,3,5-Triazin-2-yl und 1,2,4-Triazin-3-yl,
      wobei die Heterocyclen noch ein Halogenatom wie vorstehend genannt, insbesondere Fluor und Chlor, eine oder zwei C₁-C₄-Alkylgruppen wie vorstehend genannt, insbesondere Methyl oder einen Phenylrest tragen können, beispielsweise 5-Chlorbenzothiazol-2-yl, 6-Chlorpyridin-2-yl, 6-Methylpyridin-2-yl, 6-Ethylpyridin-2-yl, 6-n-Propylpyridin-2-yl, 6-Isopropylpyridin-2-yl, 6-n-Butylpyridin-2-yl, 6-sec.-Butylpyridin-2-yl und 6-tert.-Butylpyridin-2-yl, 6-Phenylpyridin-2-yl und 4,8-Dimethylchinolin-2-yl;
   besonders bevorzugt werden Halogenphenyl, C₁-C₄-Alkylphenyl, Di-(C₁-C₄)- alkylphenyl und Benzothiazol-2-yl;
R², R³
   - Wasserstoff, Cyano, Halogen wie vorstehend genannt, insbesondere Fluor und Chlor,
   - eine verzweigte oder unverzweigte C₁-C₄-Alkylgruppe wie vorstehend genannt, insbesondere Methyl, Ethyl und Isopropyl;
   - eine C₁-C₄-Alkoxygruppe wie vorstehend genannt, insbesondere Methoxy;
   besonders bevorzugt ist Wasserstoff;
R⁴, R⁵
   - Wasserstoff,
   - eine verzweigte oder unverzweigte C₁-C₄-Alkylgruppe wie vorstehend genannt, insbesondere Methyl, Ethyl, n-Propyl und n-Butyl;
   - einer der beiden Substituenten eine C₁-C₄-Alkoxygruppe wie vorstehend genannt, insbesondere Methoxy;
y
   - Sauerstoff oder Schwefel;
   - eine Gruppe -SO-, -SO₂-, -CH₂-O-SO₂-, -N=N-, -O-CO-, -CO-O- oder -CO-O-CH₂-, bevorzugt -O-CO-, -CO-O- und -CO-O-CH₂-;
   - eine C₁-C₄-Alkylenkette, die partiell oder vollständig halogeniert sein kann, insbesondere fluoriert oder chloriert, und die noch zusätzlich einen der folgenden Reste tragen kann: Cyano, Nitro, C₁-C₄-Alkyl wie vorstehend genannt, partiell oder vollständig halogeniertes C₁-C₄-Alkyl wie vorstehend genannt, C₂-C₄-Alkenyl wie vorstehend genannt, partiell oder vollständig halogeniertes C₂-C₄-Alkenyl wie vorstehend genannt, C₁-C₄-Alkoxy wie vorstehend genannt, Phenyl oder Phenoxy, wobei die letzten beiden Reste ihrerseits noch einen oder zwei der folgenden Substituenten tragen können: Cyano, Halogen wie vorstehend genannt, insbesondere Fluor und Chlor oder C₁-C₄-Alkyl wie vorstehend genannt, insbesondere Methyl; bevorzugt ist die Methylen- oder Ethylenkette;
   - eine C₂-C₄-Alkenylenkette wie Ethenylen, Prop-2-enylen und But-2-enylen, bevorzugt Ethenylen;
   - eine C₂-C₄-Alkinylenkette wie Ethinylen, Prop-2-inylen und But-2-inylen, bevorzugt Ethinylen;
   - eine Oxy- (C₁-C₄)-alkylenkette wie Oxymethylen, Oxyethylen, Oxy-n-propylen und Oxy-n-butylen, bevorzugt Oxymethylen;
   - eine Thio-(C₁-C₄)-alkylenkette wie Thiomethylen, Thioethylen, Thio-n-propylen und Thio-n-butylen, bevorzugt Thiomethylen;
   - eine C₁-C₄-Alkylenoxykette wie Methylenoxy, Ethylenoxy, n-Propylenoxy und n-Butylenoxy, bevorzugt Methylenoxy;
   - eine Carbonyl-(C₁-C₄)-alkylenkette wie Carbonylmethylen, Carbonylethylen, Carbonyl-n-propylen und Carbonyl-n-butylen, bevorzugt Carbonylmethylen;
   - eine C₁-C₄-Alkylencarbonylkette wie Methylencarbonyl, Ethylencarbonyl, n-Propylencarbonyl und n-Butylencarbonyl, bevorzugt Methylencarbonyl;
   - eine Carbonyloxy-(C₁-C₄)-alkylenkette wie Carbonyloxymethylen, Carbonyloxyethylen, Carbonyloxy-n-propylen und Carbonyloxy-n-butylen, bevorzugt Carbonyloxymethylen;
R
   - eine C₁-C₄-Alkylgruppe wie Methyl, Ethyl, n-Propyl, Isopropyl, n-Butyl, sec.-Butyl und tert.-Butyl, bevorzugt Methyl.

Besonders geeignete ortho-substituierte Phenylessigsäureamide I sind Tabelle 1 zu entnehmen, wobei Verbindungen mit R², R³ Wasserstoff, R⁴ Methyl, R⁵ Wasserstoff und R Methyl besonders bevorzugt sind. Ganz besonders gut geeignet sind 2-Methoxyimino-2-[2'-(o₋methylphenoxymethyl)-phenyl]essigsäure-N-methylamid und 2-Methoxyimino-2-(2'-(o-methylphenoxymethyl)-phenylessigsäure-N-methoxyamid.

Die Verbindungen I können bei der Herstellung als E/Z-Isomerengemische anfallen, wobei sich die beiden Isomeren durch die cis- oder trans-Stellung der Alkoxygruppe des Substituenten C=NOR zum Säureamidteil unterscheiden. Die Isomeren können gewünschtenfalls nach den hierfür üblichen Methoden, z.B. durch Kristallisation oder Chromatographie, getrennt werden. Verbindungen mit E-Konfiguration (trans-Stellung der Alkoxygruppe des Substituenten C=NOR zum Säureamidteil) sind besonders bevorzugt.

Die ortho-substituierten Phenylessigsäureamide I sind auf verschiedene Weise erhältlich, und zwar vorzugsweise nach einer der folgenden Methoden:

### a) Umsetzung von Phenylessigsäurederivaten II mit Aminen III

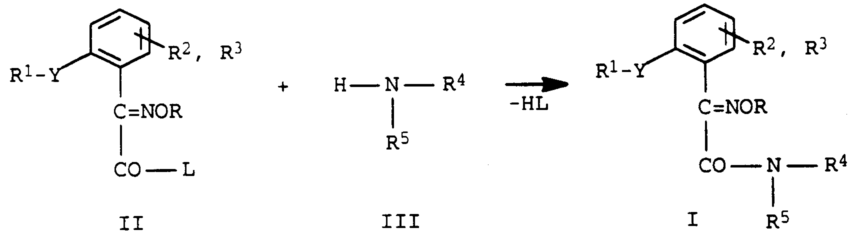

L bedeutet Halogen, insbesondere Chlor und Brom, oder C₁-C₄-Alkoxy, insbesondere Methoxy.

Zur Herstellung von ortho-substituierten Phenylessigsäureamiden I, wobei R⁴ oder R⁵ C₁-C₄-Alkoxy bedeutet, geht man vorzugsweise von den Phenylessigsäurechloriden II (L = Cl) aus.

Die Umsetzung erfolgt normalerweise nach an sich bekannten Methoden (z.B. Organikum, 16. Auflage 1985, Seiten 4O9 bis 412) in einem inerten Lösungs- oder Verdünnungsmittel, vorteilhaft in Anwesenheit einer Base.

Als Lösungs- oder Verdünnungsmittel kommen insbesondere chlorierte Kohlenwasserstoffe wie Dichlormethan, Ether wie Dioxan sowie Alkohole wie Methanol und Ethanol in Betracht.

Als Basen eignen sich beispielsweise Alkalimetallhydroxide wie Natrium- und Kaliumhydroxid, Alkalimetallcarbonate wie Natrium- und Kaliumcarbonat, Alkalimetallalkoholate wie Natriummethylat und Natriumethylat, insbesondere tertiäre Amine wie Triethylamin und heteroaromatische Amine wie Pyridin und 4-Dimethylaminopyridin. Es kann aber auch das Amin III selbst als Base verwendet werden, und zwar für eine vollständige Umsetzung in mindestens der stöchiometrischen Menge, bezogen auf die Menge an II.

Zweckmäßig setzt man alle Ausgangsverbindungen in etwa stöchiometrischem Verhältnis ein, jedoch kann in manchen Fällen auch ein Überschuß der einen oder anderen Komponente, etwa bis zu 1O mol-%, empfehlenswert sein.

Verwendet man das Amin III als Base, so liegt es in einem größeren Überschuß vor.

Im allgemeinen liegt die Reaktionstemperatur zwischen 0 und 120°C, insbesondere bei der Siedetemperatur des jeweiligen Lösungsmittels.

Bedeutet L Halogen, so ist die Durchführung der Reaktion auch in einem 2-Phasensystem unter Phasentransfer-Katalyse möglich. Dazu kann vorteilhaft eine Mischung aus einem chlorierten Kohlenwasserstoff wie Methylenchlorid, wäßriger Lauge, z.B. Natronlauge, und einem Phasentransferkatalysator wie Tetra-n-butylammoniumhydroxid verwendet werden. In diesem Fall arbeitet man z.B. bei Temperaturen zwischen 10°C und der Siedetemperatur einer der Komponenten des Lösungsmittelgemisches.

Normalerweise arbeitet man bei Atmosphärendruck. Geringerer oder höherer Druck ist möglich, bringt aber im allgemeinen keine Vorteile.

Phenylessigsäurederivate II, wobei L Halogen bedeutet, sind bekannt oder können nach bekannten Verfahren dargestellt werden (z.B. Organikum, 16. Auflage 1985, Seiten 415, 622 und 423).

Die Phenylessigsäurederivate II, wobei L C₁-C₄-Alkoxy bedeutet, sind aus den Patentanmeldungen EP-A 253 213 und EP-A 254 426 bekannt oder können nach analogen Verfahren hergestellt werden.

Beispielsweise erhält man die Phenylessigsäurederivate II mit Y = Oxymethylen, Thiomethylen oder -CO-O-CH₂- durch nucleophile Substitution an Benzylhalogeniden VI

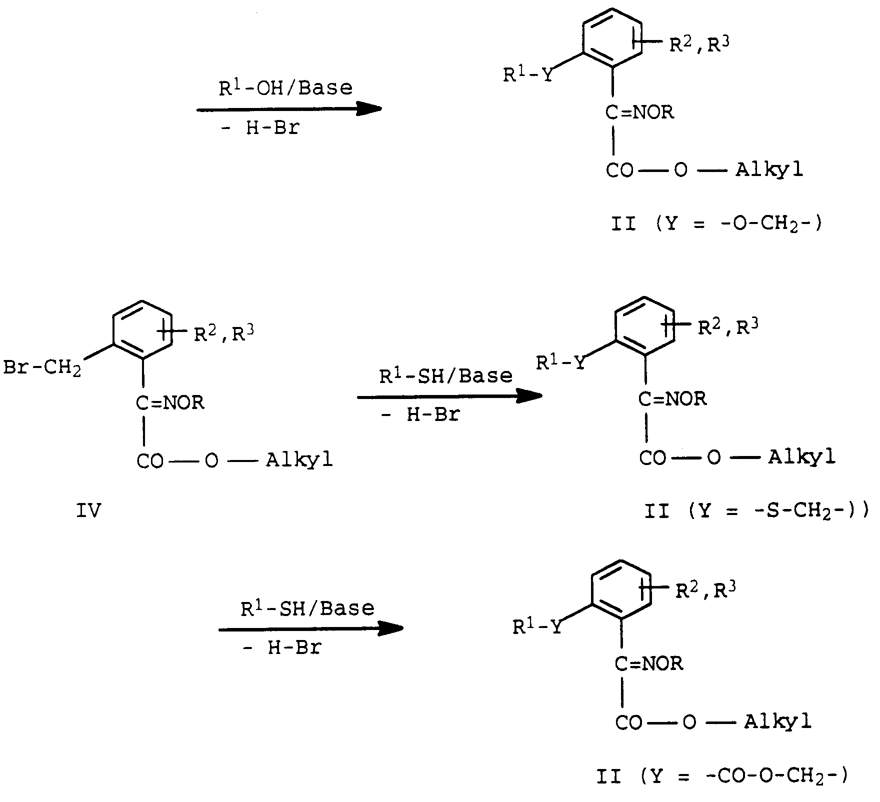

### b) Hydrolyse von Phenylacetonitrilen IV

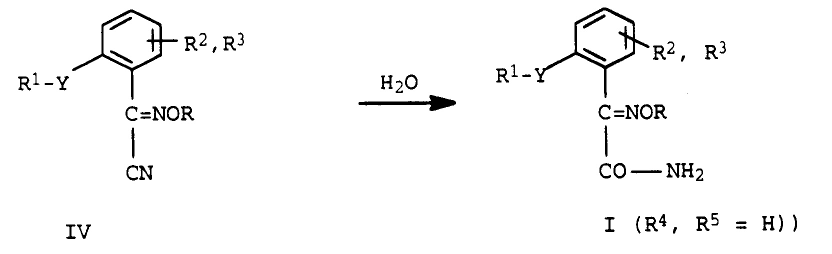

Die Hydrolyse der Phenylacetonitrile IV erfolgt normalerweise säure- oder basenkatalysiert nach an sich bekannten Methoden (vgl. z.B. Beckwith in: Zabicky "The chemistry of Amides" Seiten 119 bis 125 (1970) und Synthesis, 243 (1980)] in einem inerten Lösungs- oder Verdünnungsmittel.

Als Lösungsmittel eignen sich insbesondere Alkohole wie tert.-Butanol und Ethylenglykol.

Als Säuren kommen bevorzugt konzentrierte Mineralsäuren wie Salzsäure, Schwefelsäure und Phosphorsäure in Betracht, als Basen bevorzugt Alkalimetallhydroxide wie Natrium- und Kaliumhydroxid.

Normalerweise liegt die Reaktionstemperatur zwischen 0 und 200°C, insbesondere zwischen 20°C und der Siedetemperatur des Lösungsmittels.

Bezüglich der Mengenverhältnisse und des Druckes gelten die Angaben für Methode (a).

Die ortho-substituierten Phenylessigsäureamide I, wobei R⁴ und R⁵ Wasserstoff bedeuten, können nach an sich bekannten Verfahren [z.B. Challis in: Zabicky "The Chemistry of Amides", Seiten 731 bis 857 (1970)] am Stickstoff der Amidgruppe alkyliert werden:

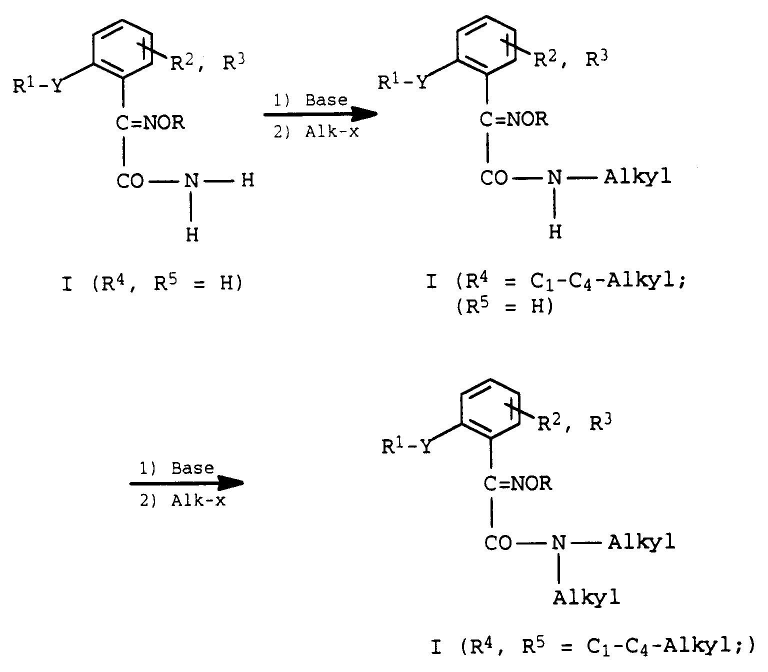

Alk bedeutet C₁-C₄-Alkyl, X bedeutet Halogen, insbesondere Brom und Jod.

Normalerweise überführt man hierzu die Phenylessigsäureamide I, wobei R⁴ und R⁵ Wasserstoff bedeuten, in einem inerten Lösungs- oder Verdünnungsmittel mittels Base in die Anionen und setzt diese mit einem Alkylhalogenid, bevorzugt einem Alkyljodid, um.

Als Lösungs- oder Verdünnungsmittel kommen bevorzugt Ether wie Tetrahydrofuran und Dioxan in Betracht.

Als Basen eignen sich insbesondere Alkalimetallhydroxide wie Natrium- und Kaliumhydroxid sowie Alkalimetallhydride wie Natrium und Kaliumhydrid.

Im allgemeinen liegt die Reaktionstemperatur zwischen 0 und 100°C, insbesondere bei der Siedetemperatur des jeweiligen Lösungsmittels.

Bezüglich der Mengenverhältnisse und des Druckes gelten die Angaben für Methode (a).

Die ortho-substituierten Phenylessigsäureamide I eignen sich als Fungizide und zur Bekämpfung von Schädlingen wie Insekten, Nematoden und Akaridien.

Die ortho-substituierten Phenylessigsäureamide I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophtora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Die ortho-substituierten Pgenylessigsäureamide I eignen sich des weiteren zur Bekämpfung von Schädlingen aus der Klasse der Insekten, Spinnentiere und Nematoden. Sie können im Pflanzenschutz sowie auf dem Hygiene-, Vorratsschutz- und Vetrinärsektor als Schädlingsbekämpfungsmittel eingesetzt werden.

Zu den schädlinchen Insekten gehören:
- aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria buoliana, Feltia subterranea, Galleria mellonella, Grapholitha funebrana, Grapholitha malesta, Heliothis armigera, Heliothis virescens, Heliothis zea, Hellula undalis, Hibernia defoliaria, Hyphantria cunea, Hyponomeuta malinellus, Keifferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostriania nubilalis, Panolis flamea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocama, Tortrix viridana, Trichoplusia ni und Zeiraphera canadensis;
- aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus leantis, Byctiscus betulae, Cassida nebulosa, Cerotoma trifurcata, Ceuthorrhynchus assimilis, Ceuthorrynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Otiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus und Sitophilus granaria;
- aus der Ordnung der Zweiflügler (Diptera) beispielsweise Aedes argypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipieans, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Musciana stabulans, Oestrus ovis, Oscinella frit, Pegomya hyoscyami, Phorbia antiqua, Phorbia brassicae, Phorbia coarctata, Rhagoletis cerasi, Rhagoletis pomonella, Tabanus bovinus, Tipula oleracea, und Tipula paludosa;
- aus der Ordnung der Thripse (Thysanoptera) beispielsweise Frankliniella fusca, Frankliniella occidentalis, Frankliniella tritici, Scirtothrips citri, Thrips oryzae, Thrips palmi und Thrips tabaci;
- aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata und Solenopsis invicata;
- aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis und Thyanta perditor;
- aus der Ordnung der Pflanzensauger (Homoptera) beispielsweise Acyrthosiphon onobrychis, Adelges laritis, Aphidula nasturtii, Aphis fabae, Aphis pomi, Aphis sambuci, Brachycaudus cardui, Brevicoryne brassicae, Cerosipha gossypii, Dreyfusia normannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mali, Schizaphis graminum, Schizoneura lanuginosa, Trialeurodes vaporariorum und Viteus vitifolii;
- aus der Ordnung der Termiten (Isoptera) beispielsweise Calotermes flavicollis, Leucotermes flavipes, Reticulitermes lucifugus und Termes natalensis;
- aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplanata americana, Schistocerca americana, Schistocerca peregrina, Stauronotus maroccanus und Tachycines asynamorus;
- aus der Klasse der Arachnoidea beispielsweise Spinnentiere (Acarina) wie Amglyomma americanum, Amglyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoani, Hyalorna truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius und Tetranychus urticae;
- aus der Klasse der Nematoden beispielsweise Wurzelgallennematoden, z.B. Meloidogyne hapla, Meloidogyne incognita, Meloidogyne javaniva, Zysten-bildende Nematoden, z.B. Globodera rostochiensis, Heterodera avenae, Heterodera glycine, Heterodera schachtii, Heterodera trifolii, Stock- und Blattälchen, z.B. Belonolaimus longicaudatus, Ditylenchus destructor, Ditylenchus dipsaci, Helicotylenchus multicinctus, Longidorus alongatus, Radopholus similis, Rotenchus robustus, Trichodorus primitivus, Tylenchorhynchus claytoni, Tylenchorhynchus dubius, Pratylenchus neglectus, Pratylenchus penetrans, Pratylenchus curvitatus und Pratylenchus goodeyi.

Die Wirkstoffe können in die üblichen Formulierungen übergeführt werden, wie Lösungen, Emulsionen, Suspensionen, Stäube, Pulver, Pasten und Granulate. Die Anwendungsformen richten sich nach den Verwendungszwecken; sie sollen in jedem Fall eine feine und gleichmäßig Verteilung des ortho-substituierten Phenylessigsäureamids gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsnittel verwendet werden können.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tterischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, öldispersionen) durch Zusatz von Wasser bereitet werden. Zur Herstellung von Emulsionen, Pasten oder öldispersionen können die Substanzen als solche oder in einem Öl oder Lösungsmittel gelöst, mittels Netz-, Haft-, Dispergier- oder Emulgiermitttel in Wasser homogenisiert werden.

Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensatioansprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin-Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Wirkstoffkonzentrationen in den anwendungsfertigen Zubereitungen können in größeren Bereichen variiert werden.

Ganz allgemein enthalten die Mittel zwischen 0,0001 und 95, vorzugsweise zwischen 0,01 und 90 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Beispiele für solche Zubereitungen sind:
I. eine Lösung aus 90 Gew.-Teilen einer erfindungsgemäßen Verbindung und 10 Gew.-Teilen N-Methyl-α-pyrrolidon, die zur Anwendung in Form kleinster Tropfen geeignet ist;
II. eine Mischung aus 20 Gew.-Teilen einer erfindungsgemäßen Verbindung, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.- Teilen Calciumsalz der Dodecylbenzolsulfonsäure, 5 Gew.-Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen einer erfindungsgemäßen Verbindung, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen einer erfindungsgemäßen Verbindung, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen einer erfindungsgemäßen Verbindung, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung, 10 Gew.-Teilen des Natriumsalzes eines Phenolsulfonsäureharnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd-Kondensates und 68 Gew.-Teilen eines paraffinischen Mineralöls.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Aufwandmengen in fungiziden Mitteln liegen je nach Art des gewünschten Effektes zwischen 0,02 und 3 kg Wirkstoff pro ha. Die neuen Verbindungen können auch im Materialschutz (Holzschutz) eingesetzt werden, z.B. gegen Paecilomyces variotii.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Insekten beträgt unter Freilandbedingungen 0,02 bis 10, vorzugsweise 0,1 bis 2,0 kg/ha.

Die erfindungsgemäßen Mittel können in diesen Anwendungsformen auch zusammen mit anderen Wirkstoffen vorliegen, z.B. mit Herbiziden, Insektiziden, Wachstumsregulatoren, Fungiziden oder auch mit Düngemitteln. Diese Mittel können zu den erfindungsgemäßen Mitteln im Gewichtsverhältnis 1 : 10 bis 10 : 1, gegebenenfalls auch erst unmittelbar vor der Anwendung (Tankmix), zugesetzt werden. Beim Vermischen mit Fungiziden oder insektiziden erhält man dabei in vielen Fällen eine Vergrößerung des Wirkungsspektrums.

Die Mittel bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

### Herstellungsbeispiele

### Beispiel 1

### 2-Methoxyimino-2-[2'-(o,p-dimethylphenoxymethyl)-phenyl]-essigsäure-N-methylamid

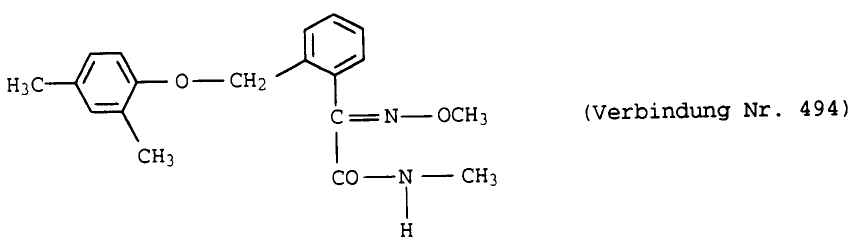

0,465 g (15 mmol) über Kaliumhydroxid getrocknetes Monomethylamin wurden bei etwa 25°C in eine Lösung aus 5,0 g (15 mmol) 2-Methoxyimino-2-[2'-(o,p-dimethylphenoxymethyl)]-phenylessigsäurechlorid in 30 ml Dichlormethan eingegast. Diese Mischung wurde eine Stunde gerührt und anschließend mit 70 ml Dichlormethan verdünnt. Nach Extraktion von Nebenprodukten mit 100 ml Wasser arbeitete man die organische Phase wie üblich auf das Produkt hin auf. Ausbeute: 88 % (Öl);
1H-NMR (in CDCl₃, TMS als Standard): d = 2.20(s,3H); 2.25(s'3H); 2.90(d,3H); 3.94(s,3H); 4.93(s,2H); 6.70-7.60(m,7H).

### Beispiel 2

### 2-Methoxyimino-2-£2'-(o,p-dimethylphenoxymethyl)-phenyl)-essigsäure-N,N-dimethylamid

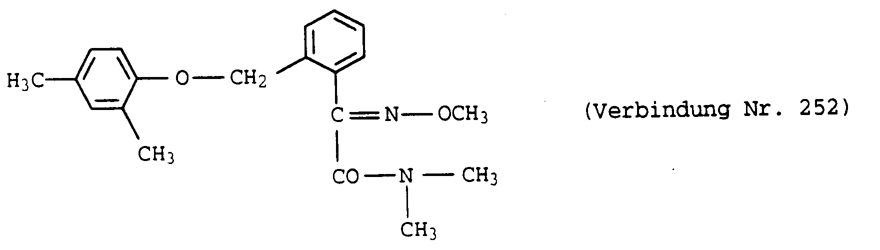

Analog Beispiel 1 wurden 0,675 g (15 mmol) getrocknetes Dimethylamin mit 5,0 g (15 mmol) 2-Methoxyimino-2-(2'-(o,p-dimethylphenoxymethyl)]-phenyl-essigsäurechlorid umgesetzt. Ausbeute: 78% (Öl);
¹H-NMR (in CDCI₃, TMS als Standard): d = 2.20(5,3H); 2.23(s,3H) 3.02(5,3H); 3.18(s,3H); 3.95(s,3H); 5.02(s,2H); 6.60-7.60(m,7H).

In Tabelle 1 sind noch weitere Endprodukte I aufgeführt, welche auf die gleichen Weisen hergestellt wurden oder herstellbar sind.

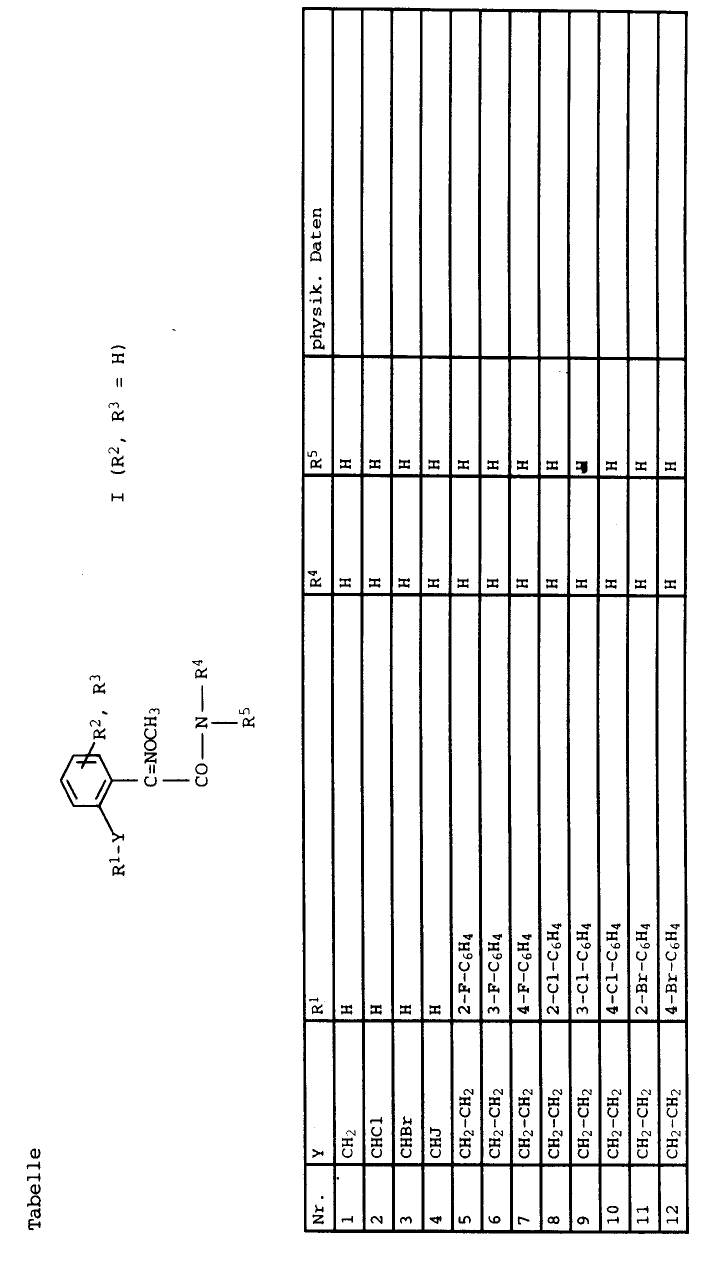

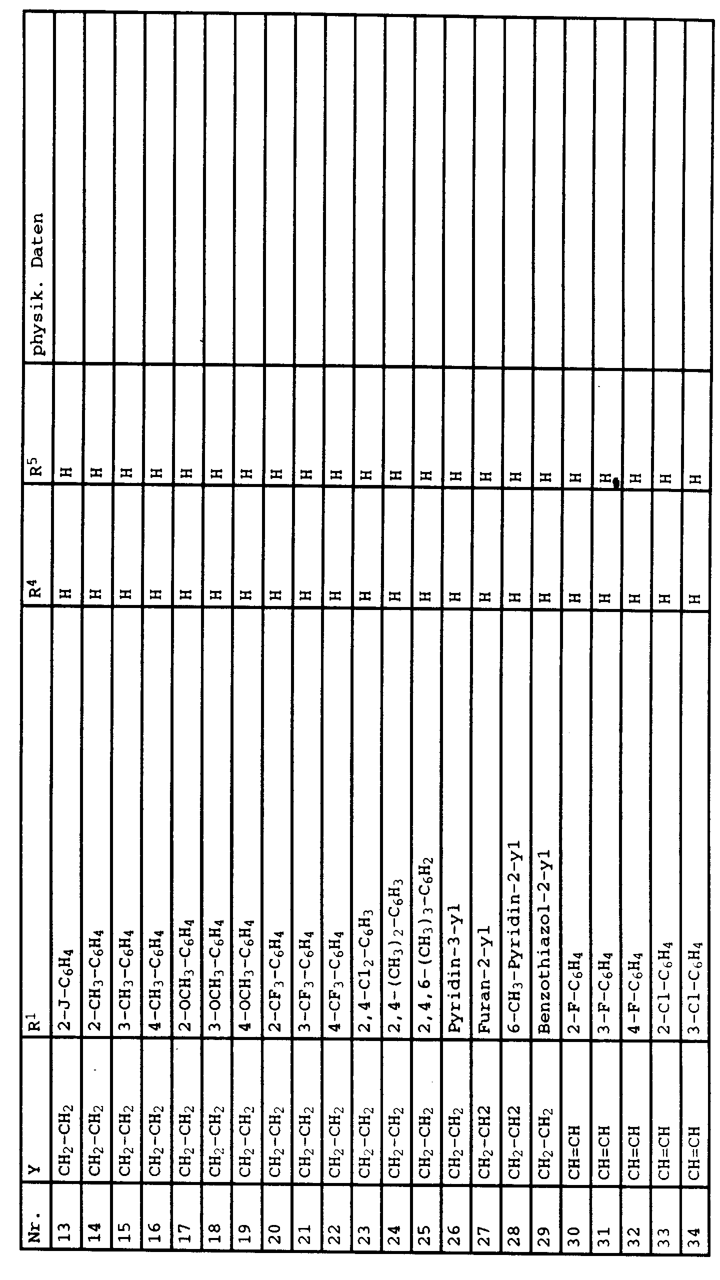

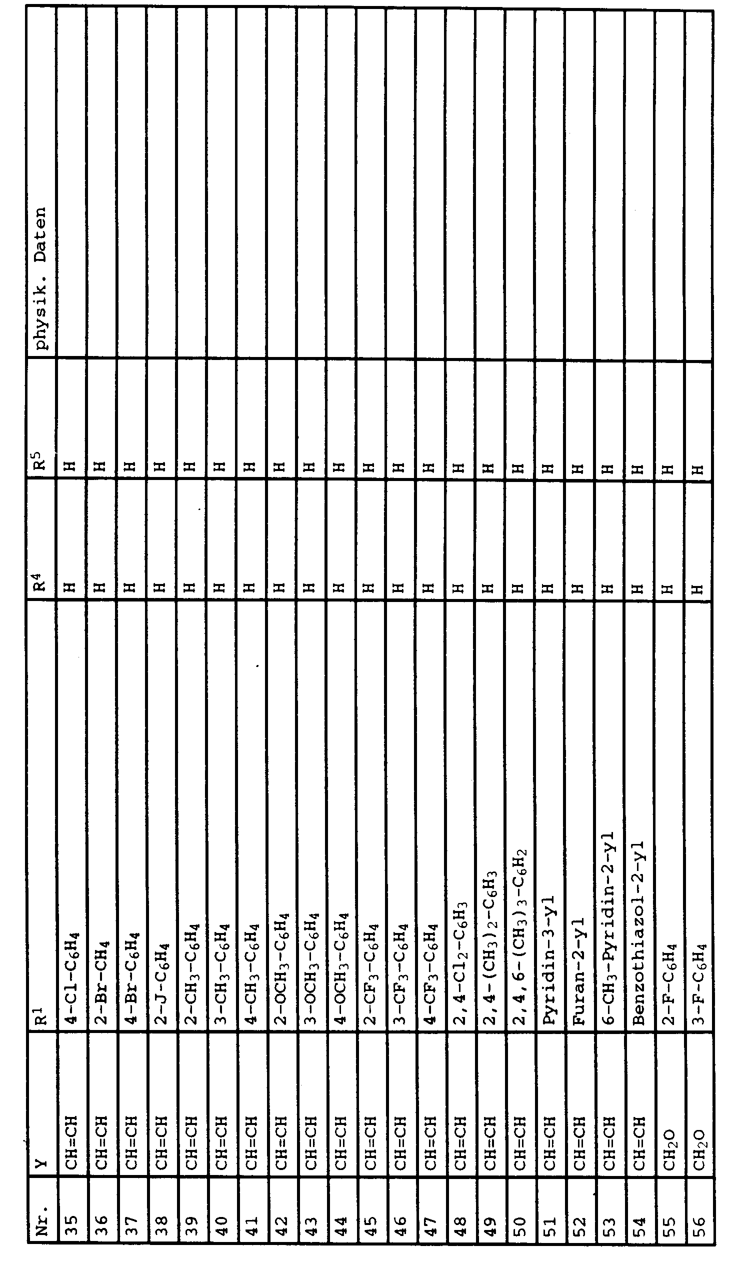

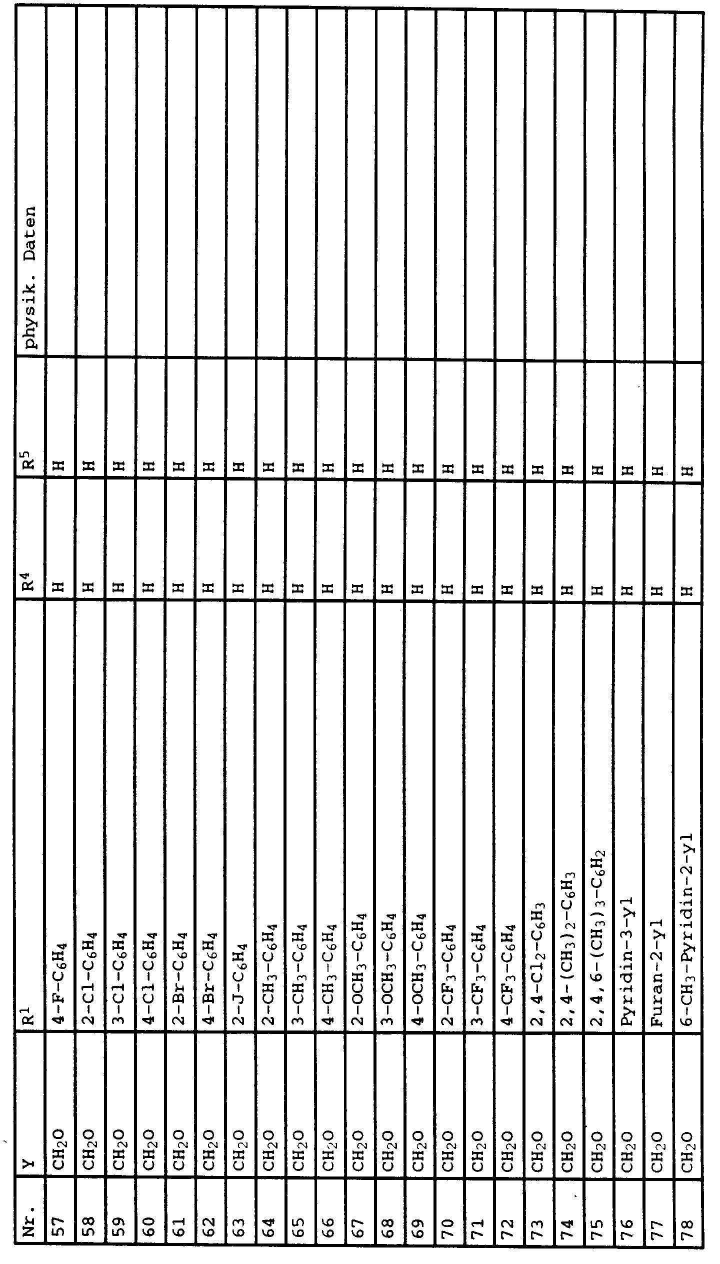

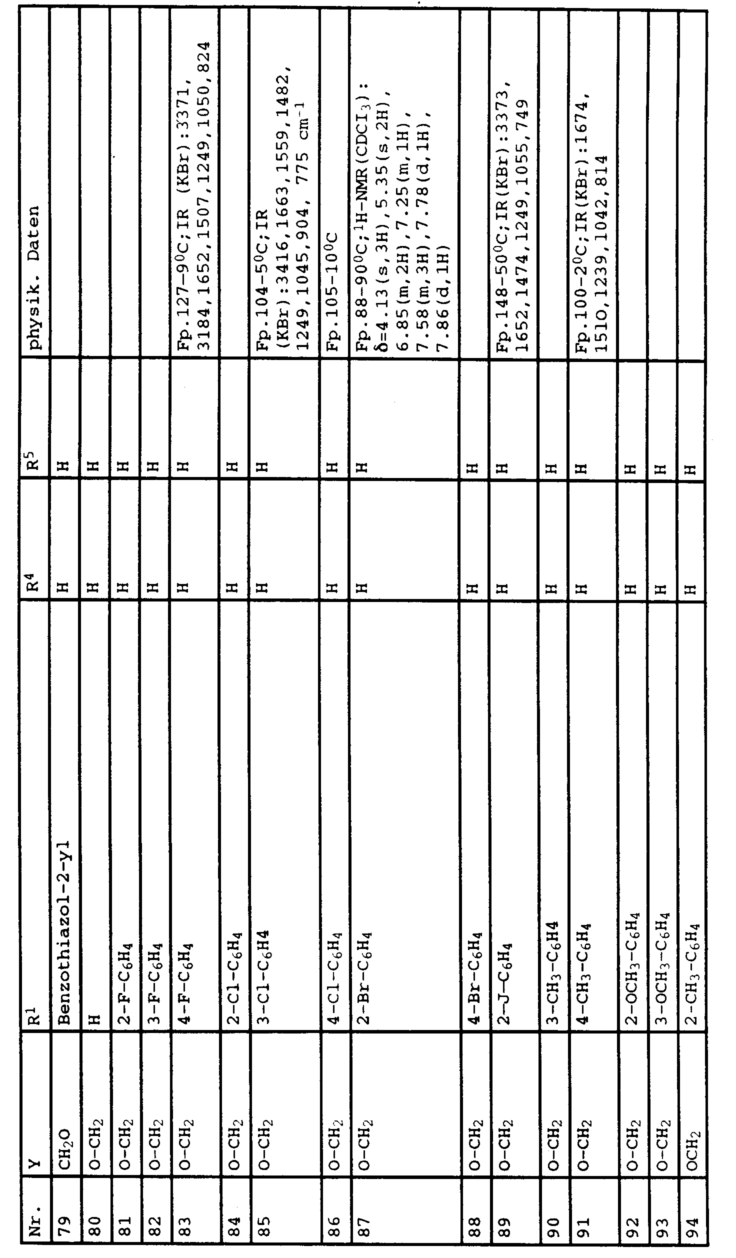

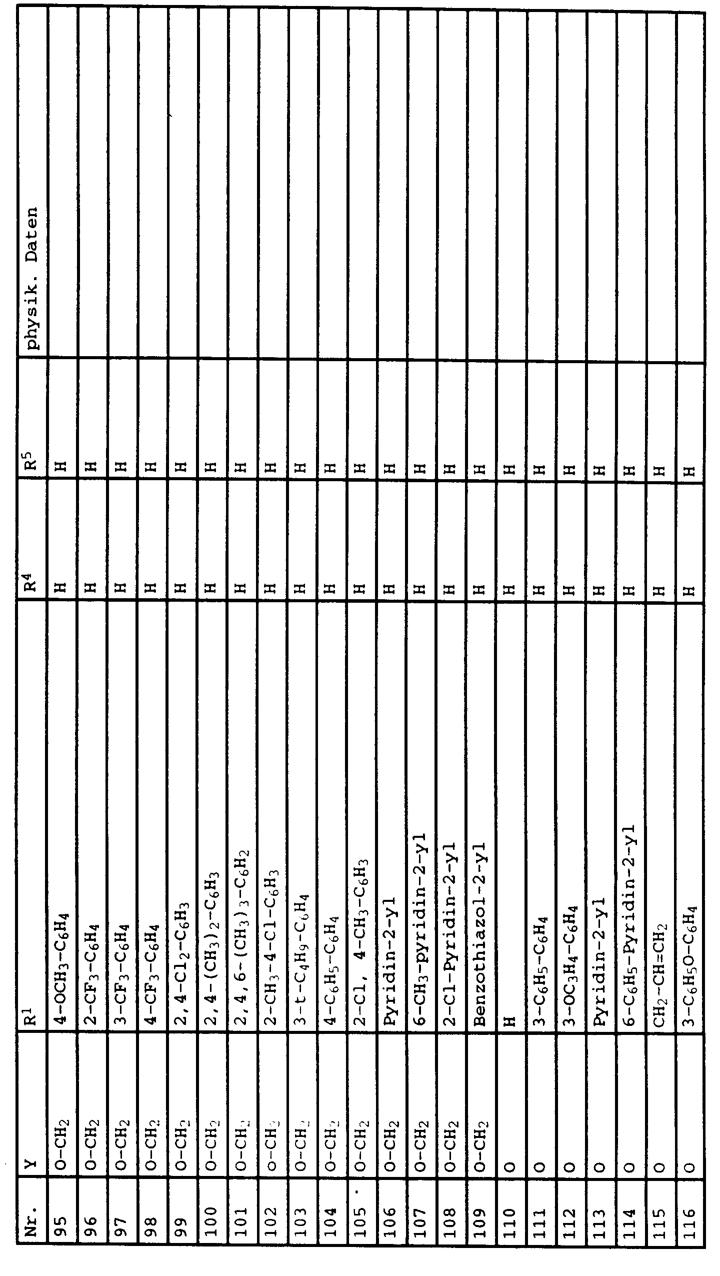

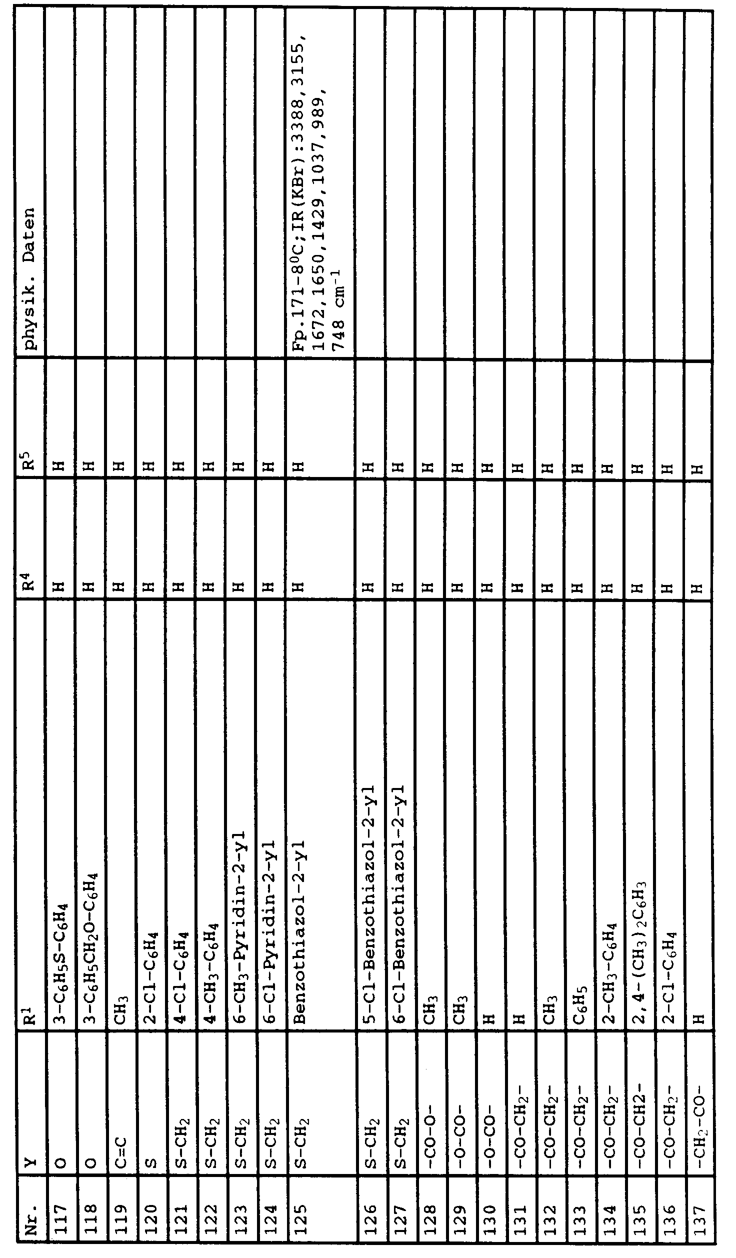

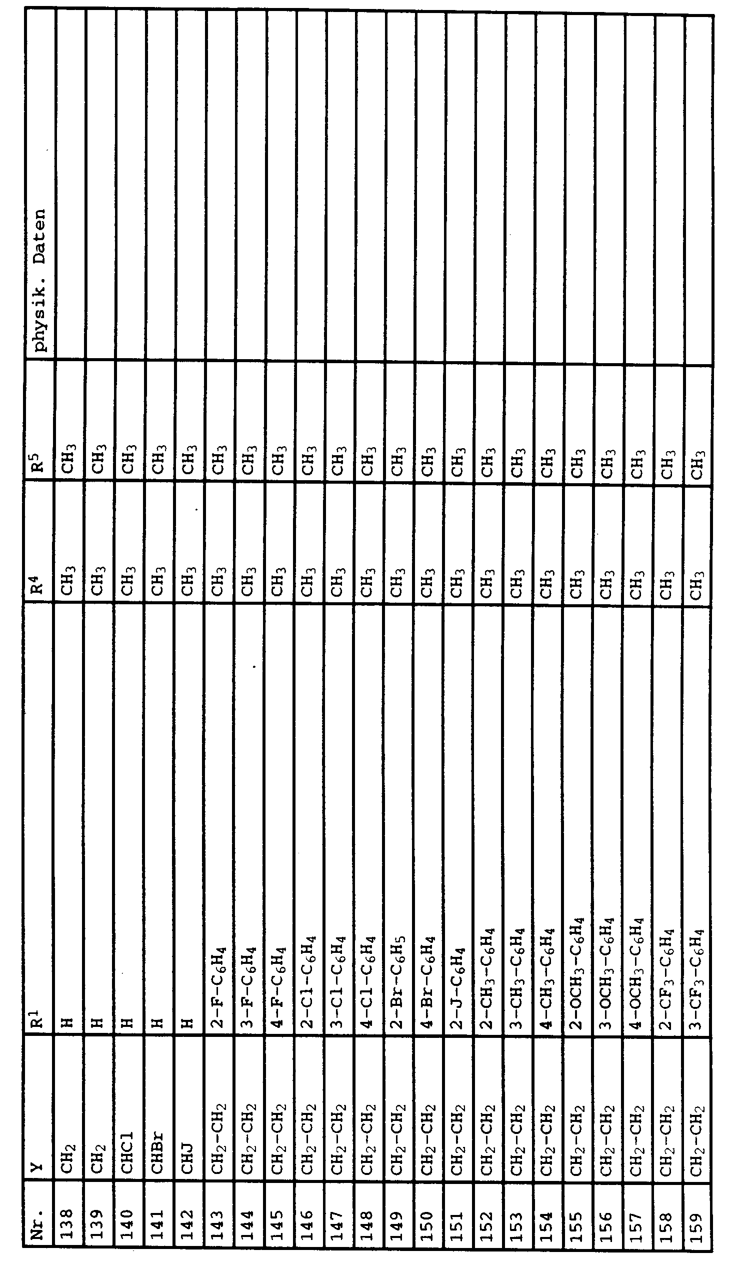

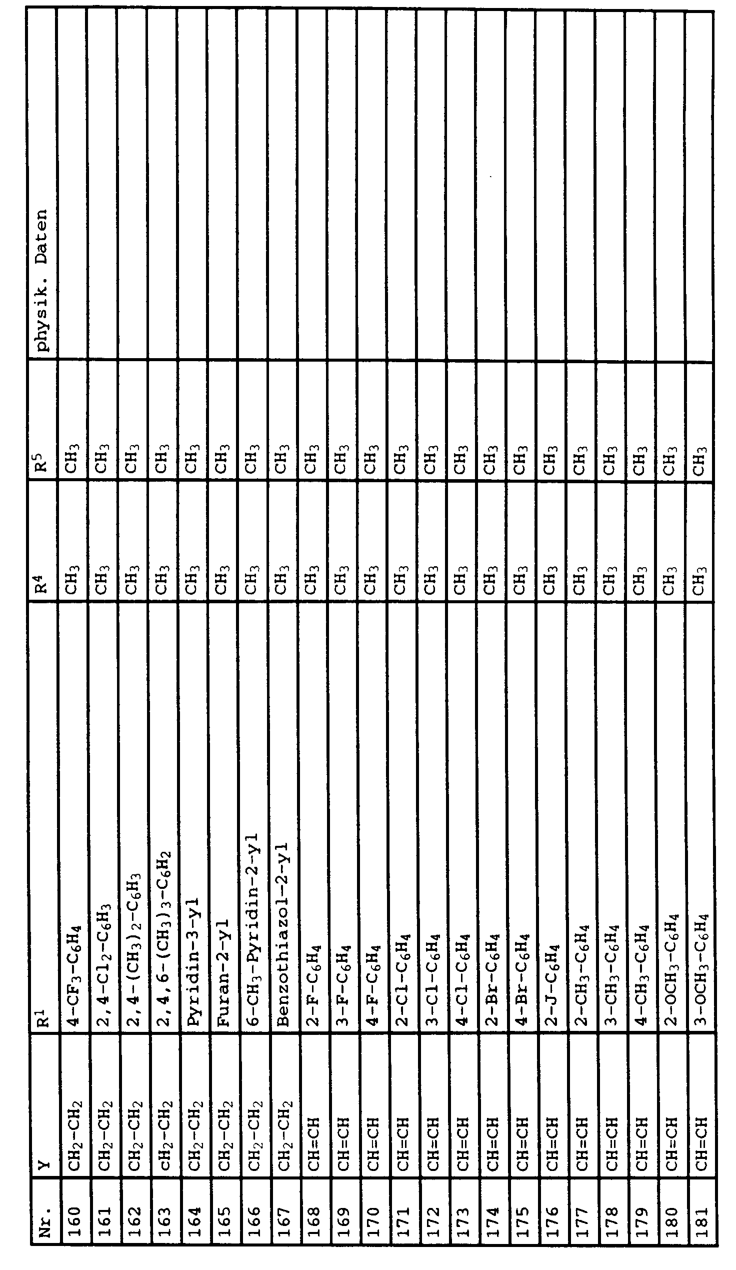

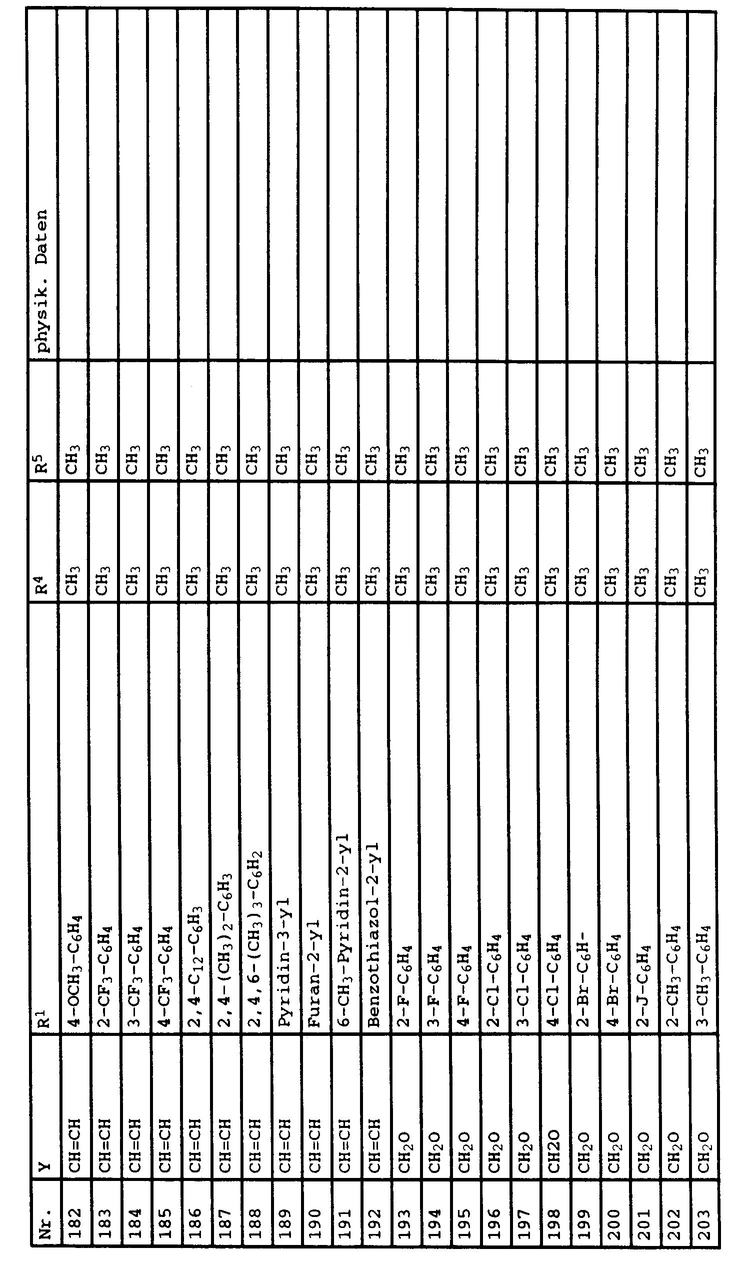

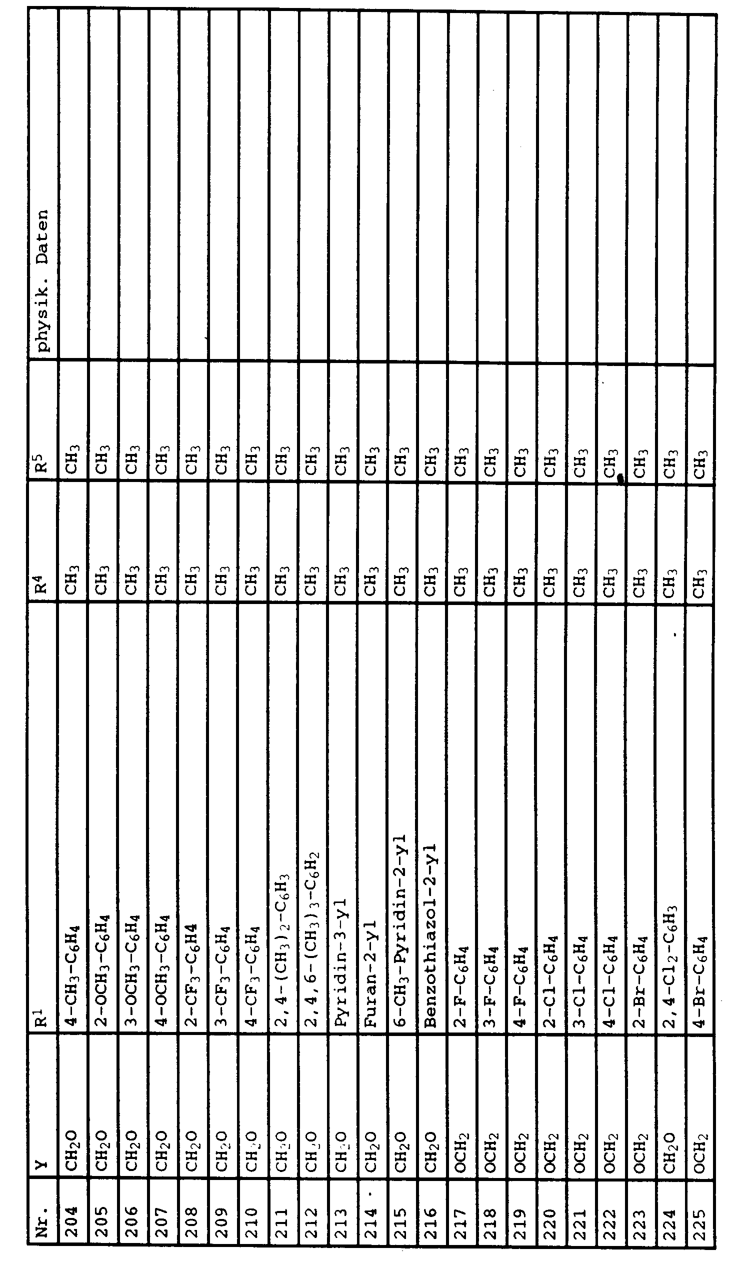

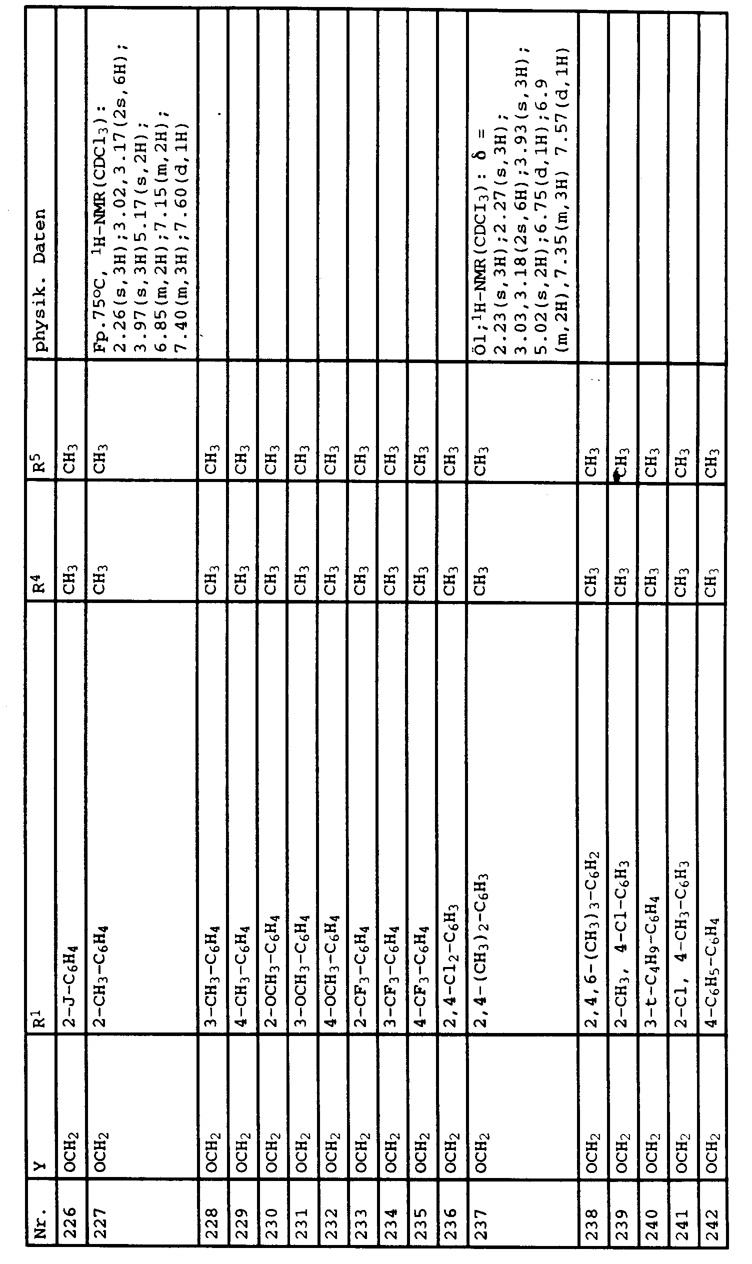

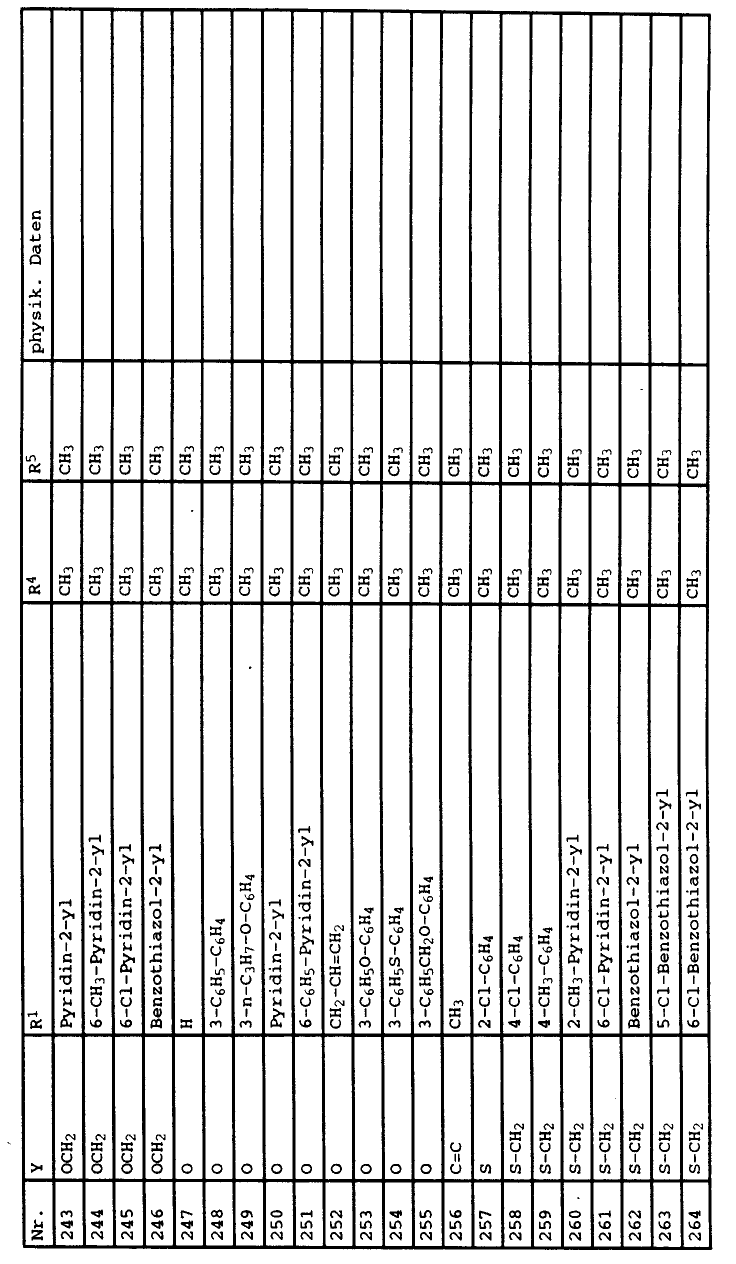

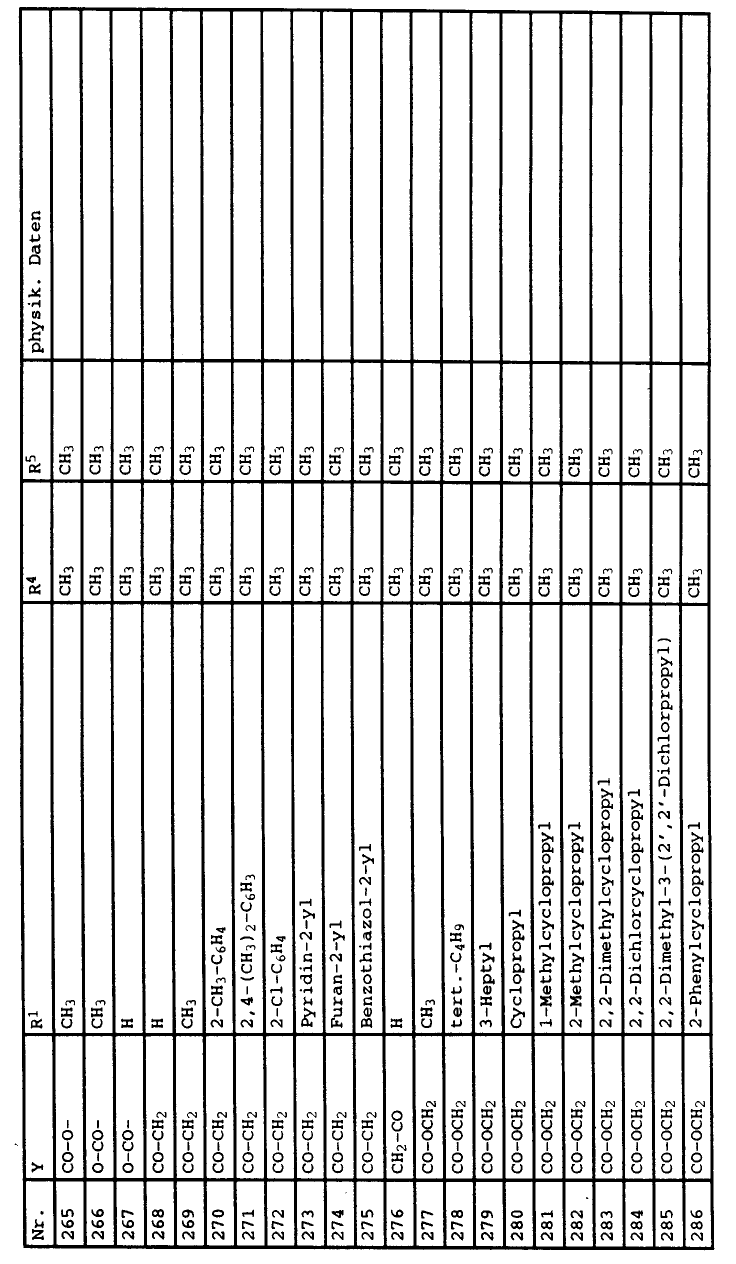

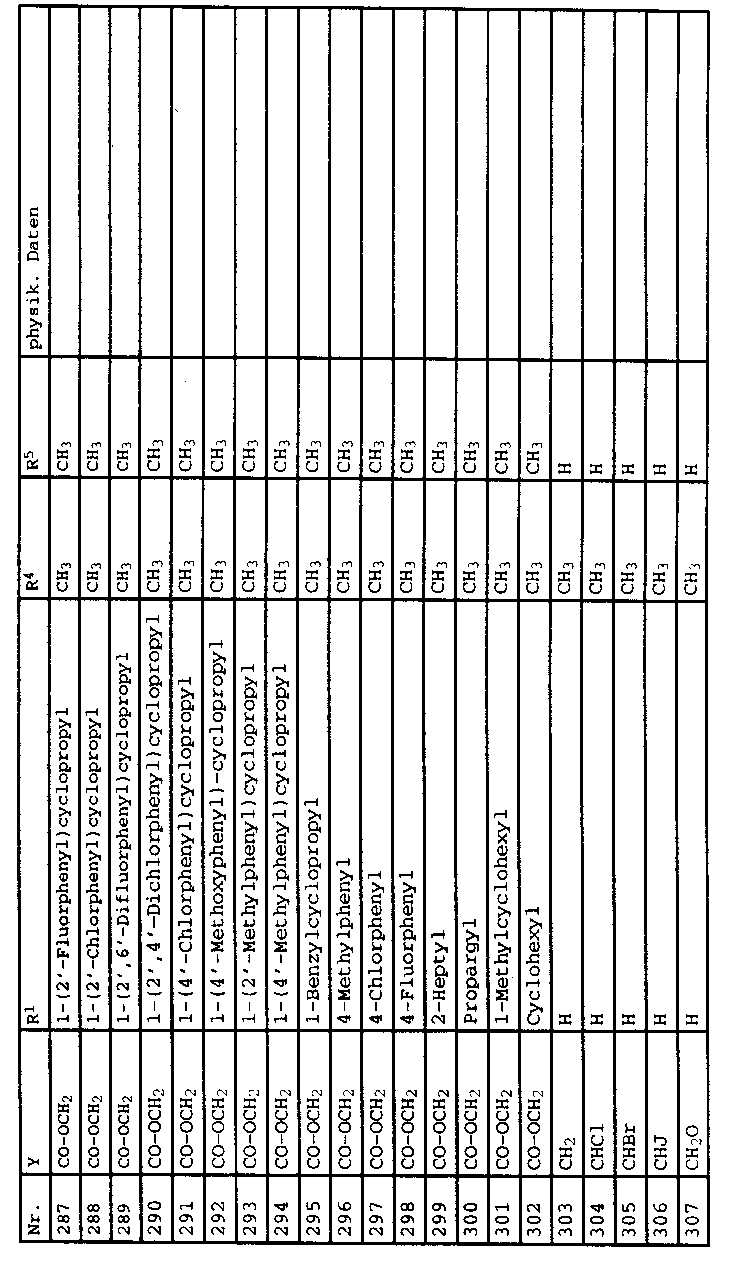

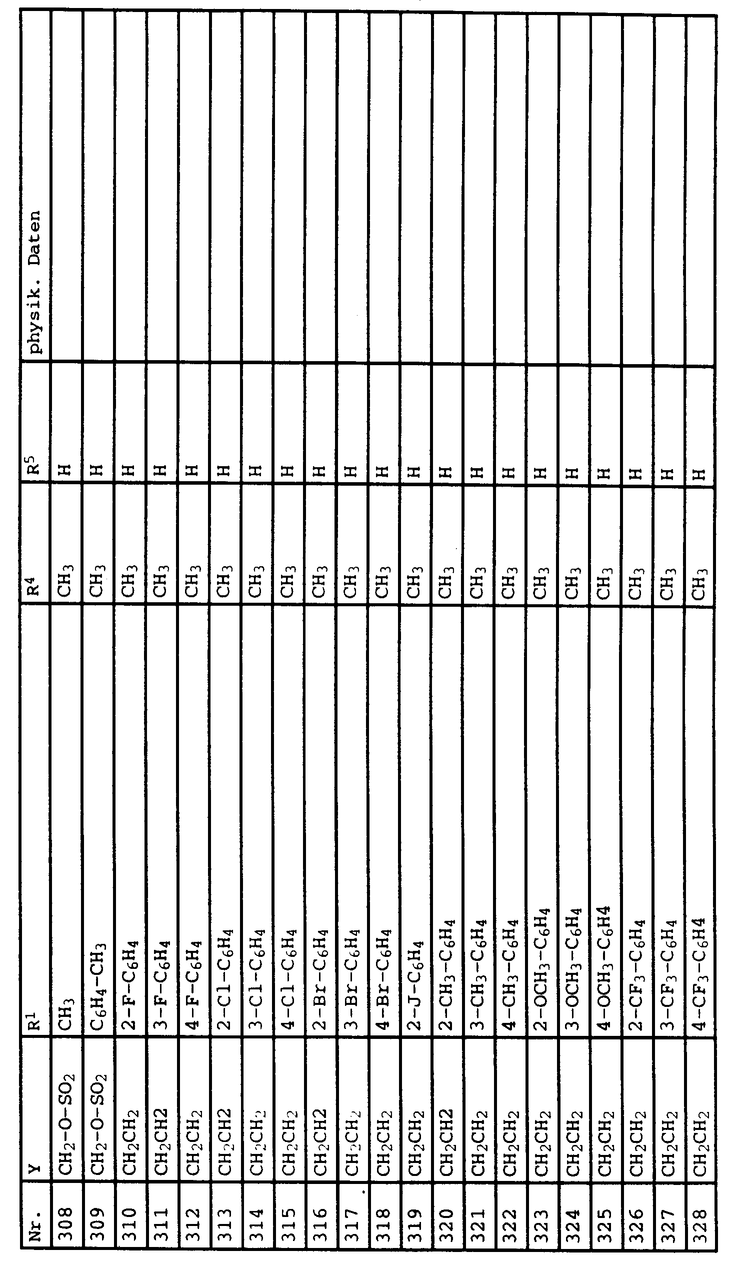

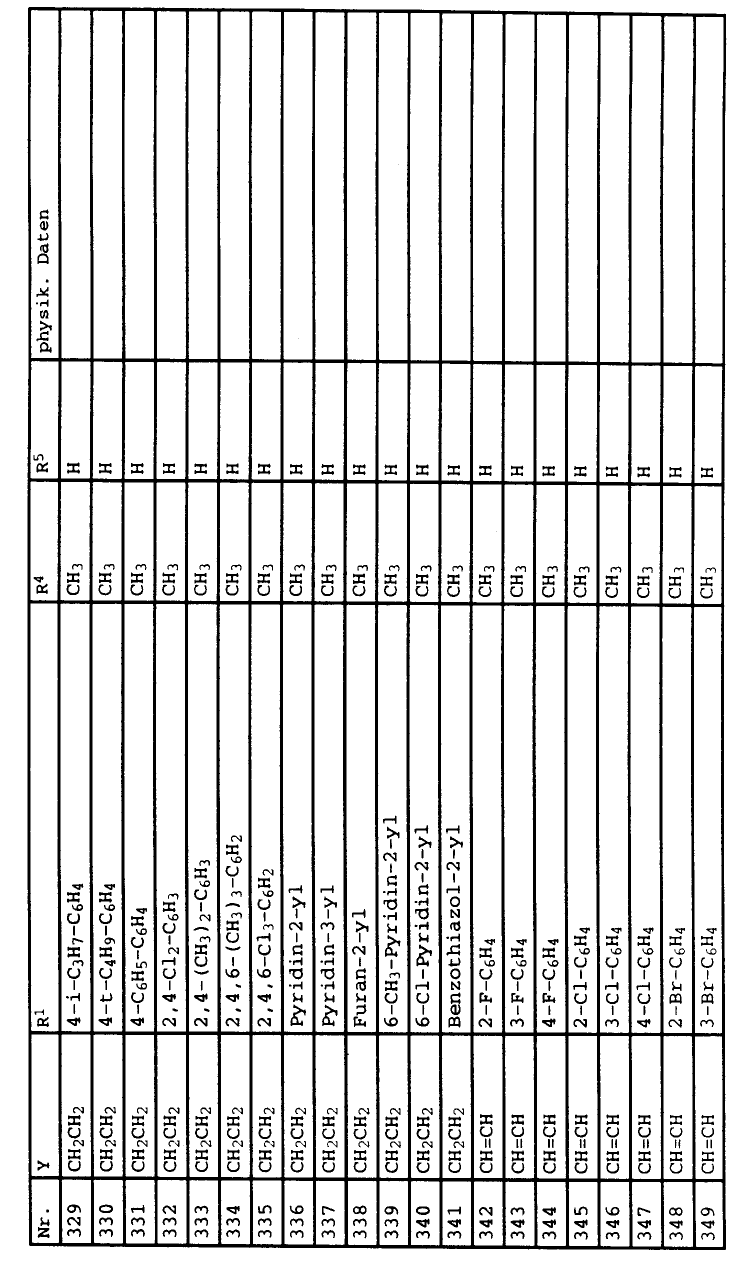

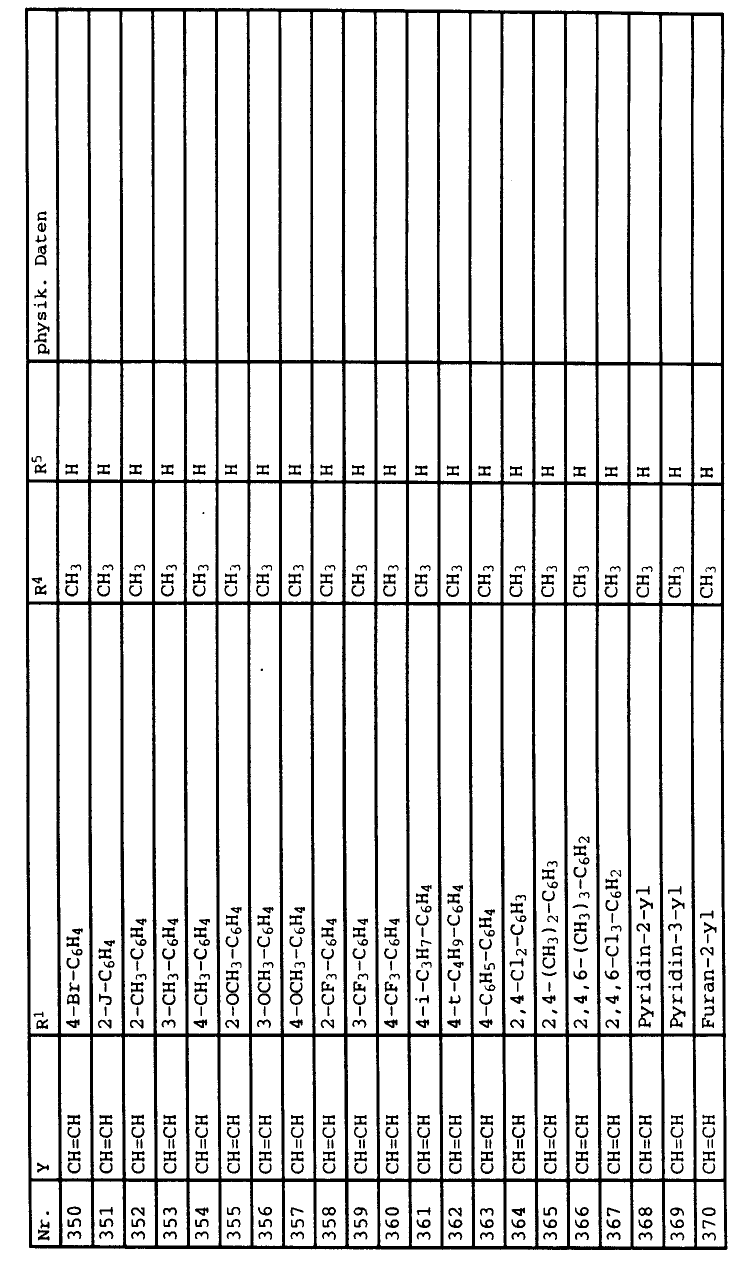

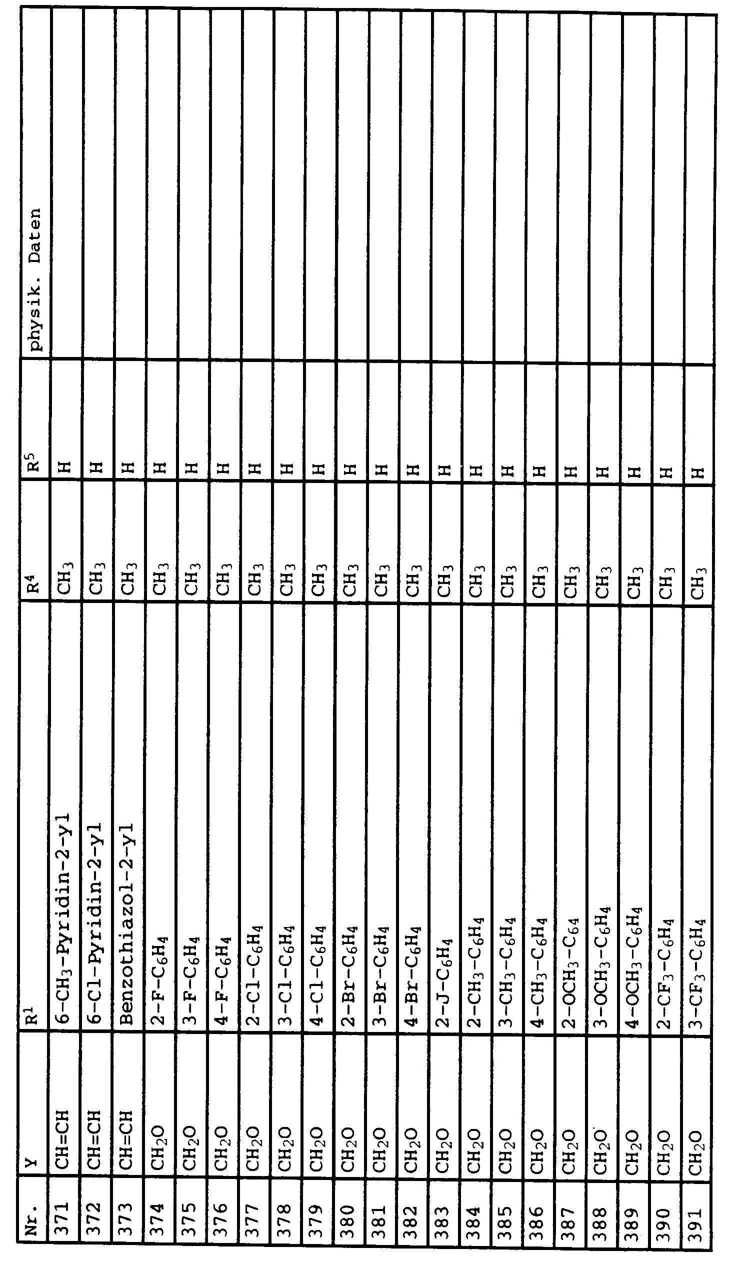

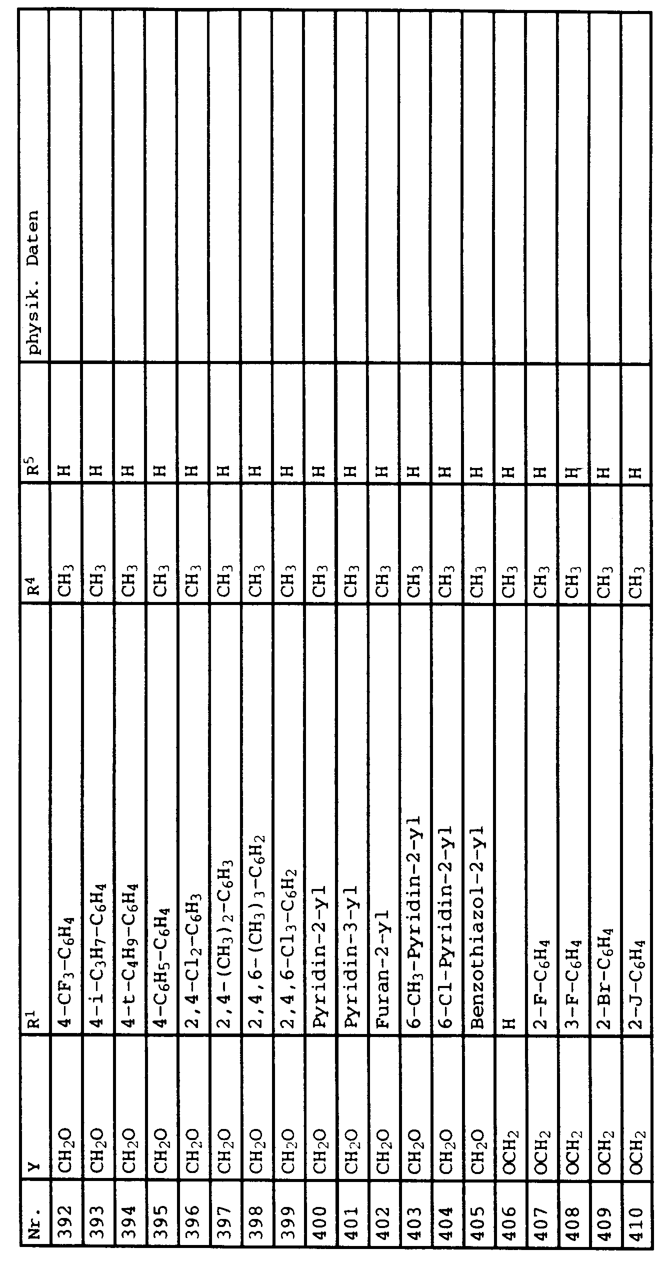

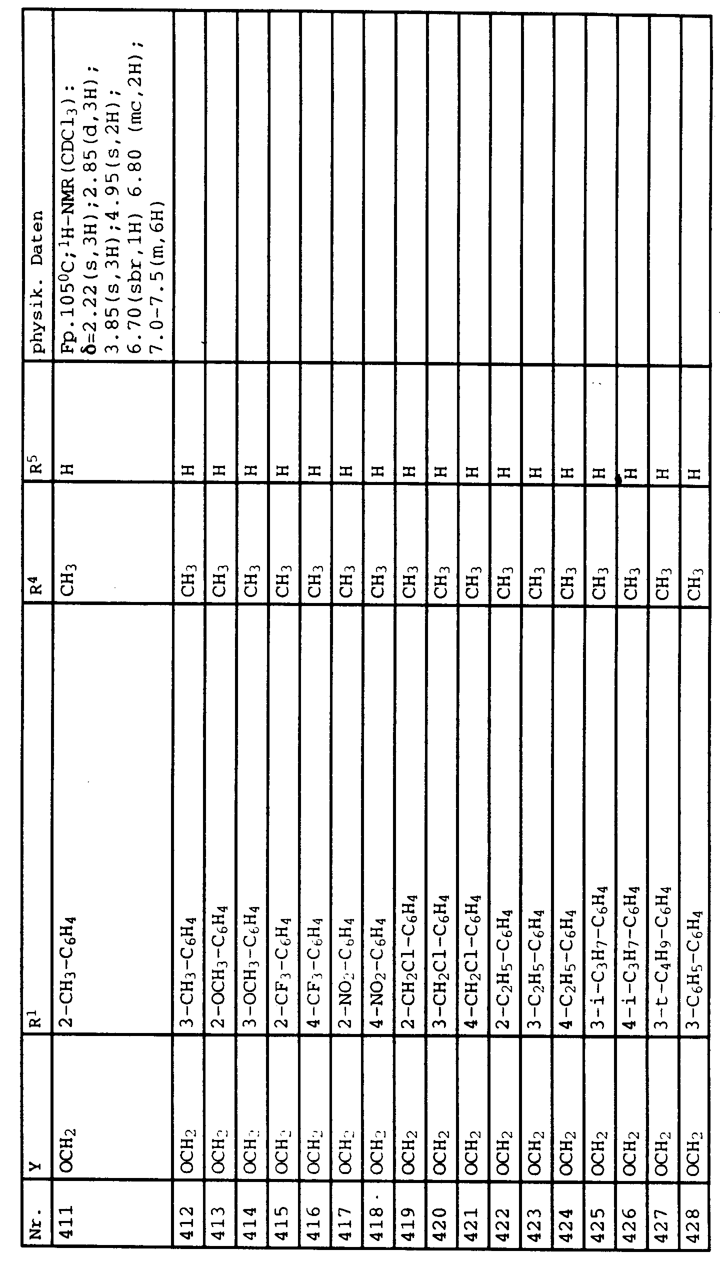

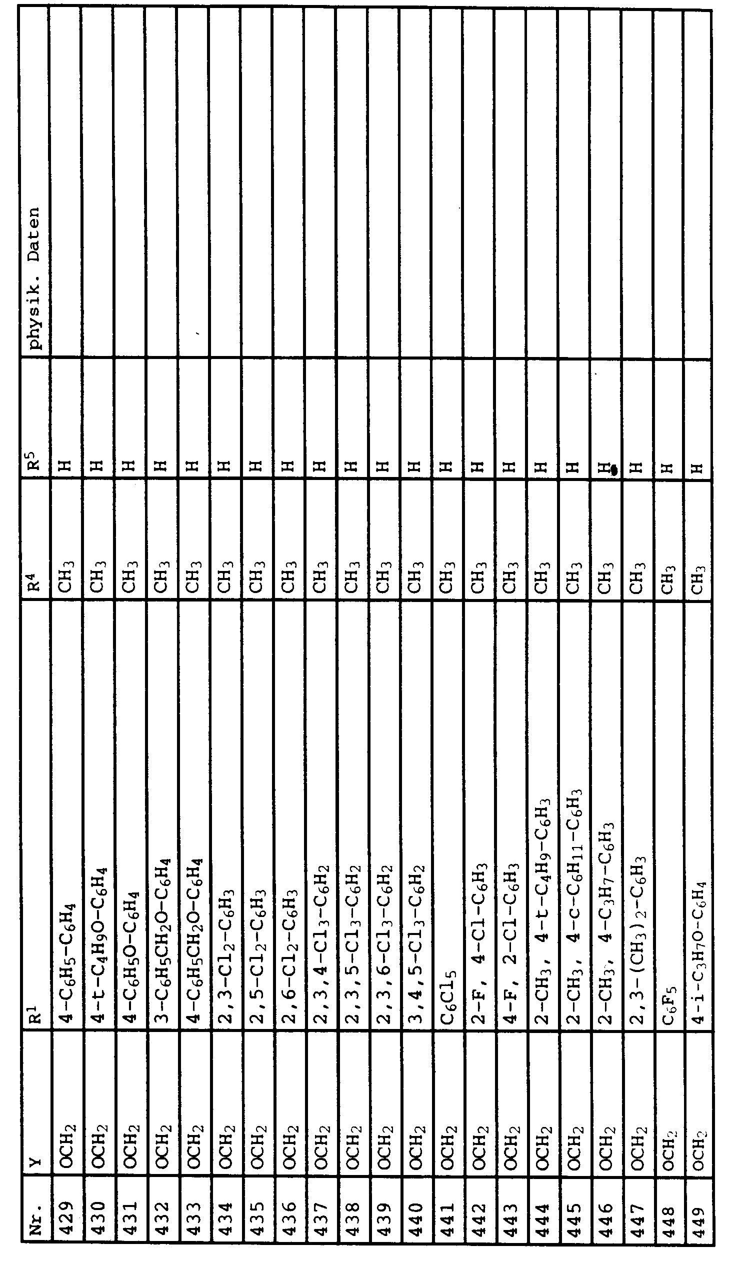

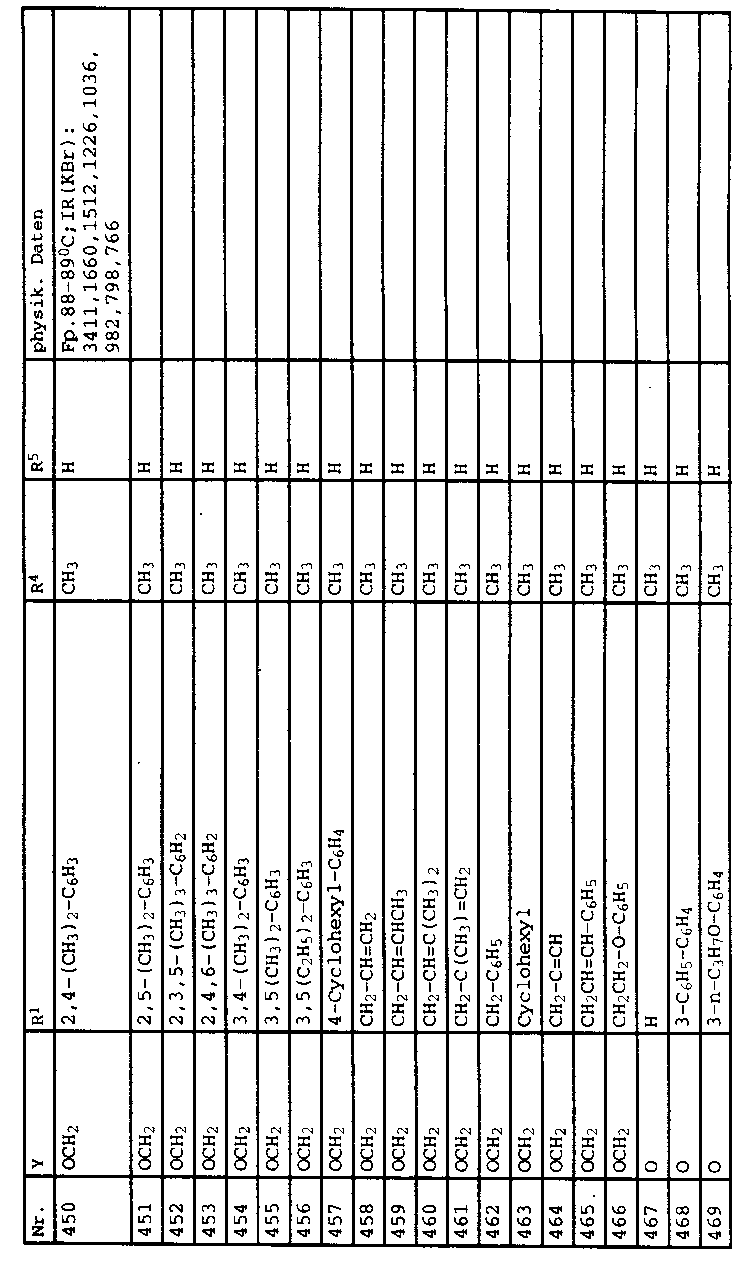

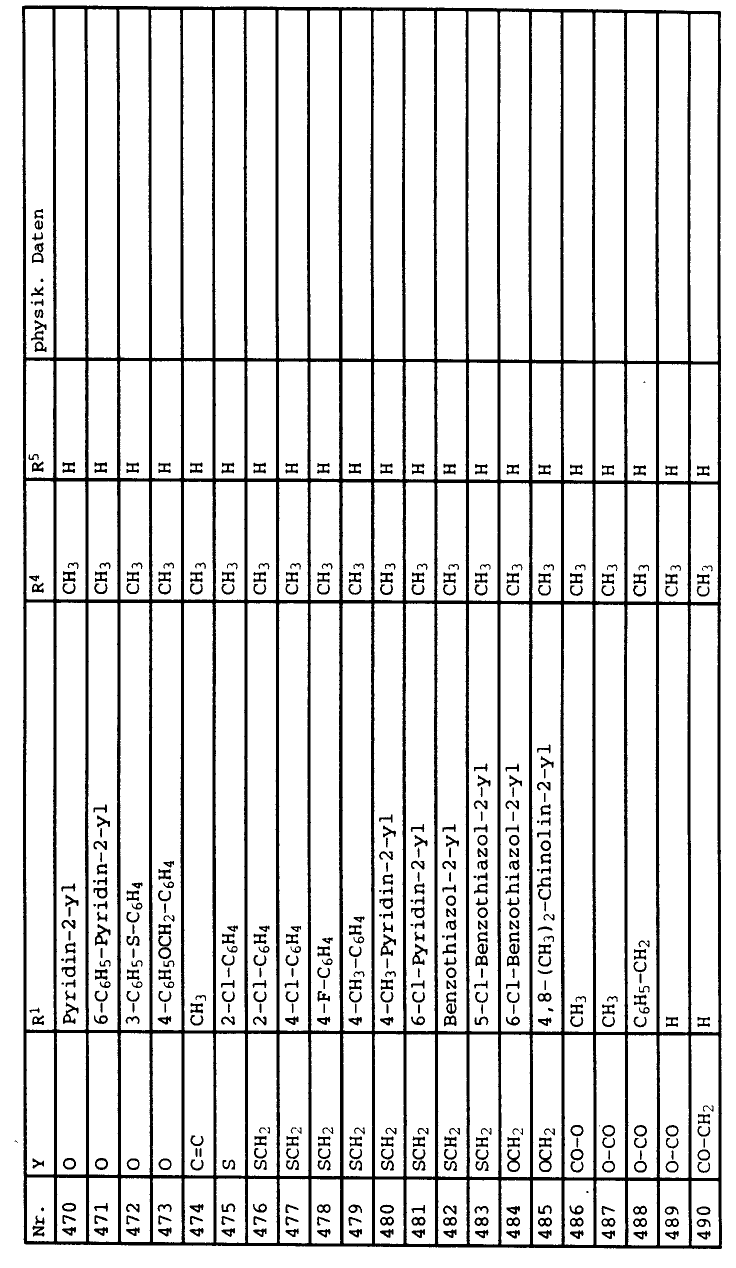

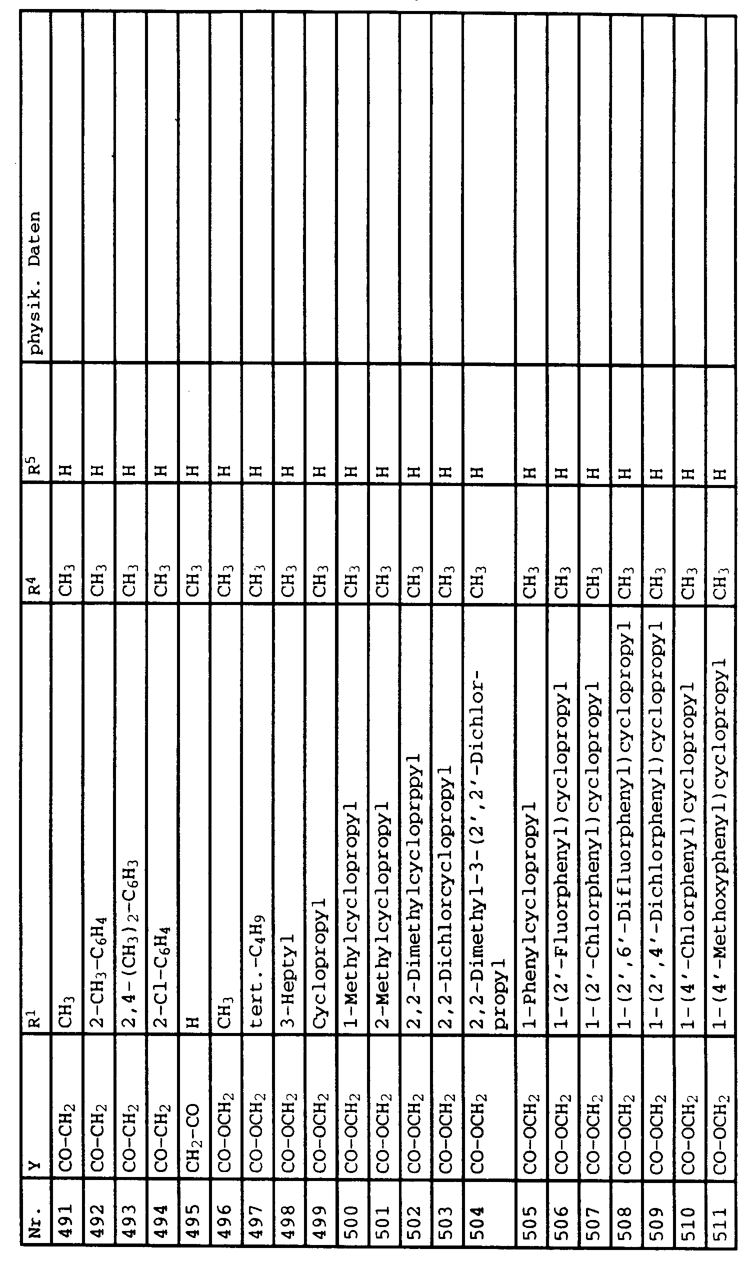

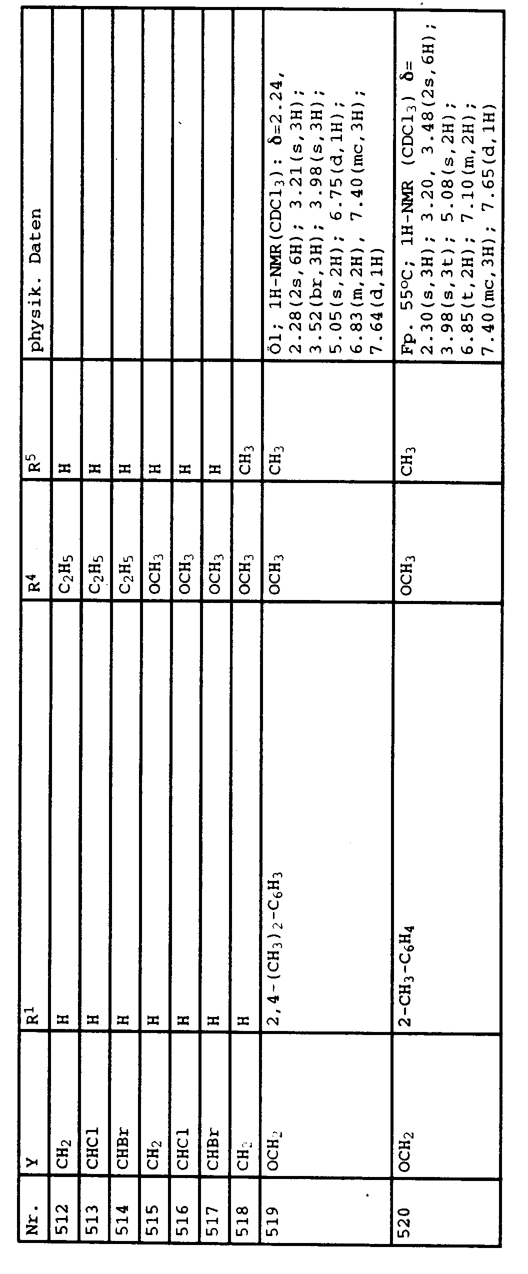

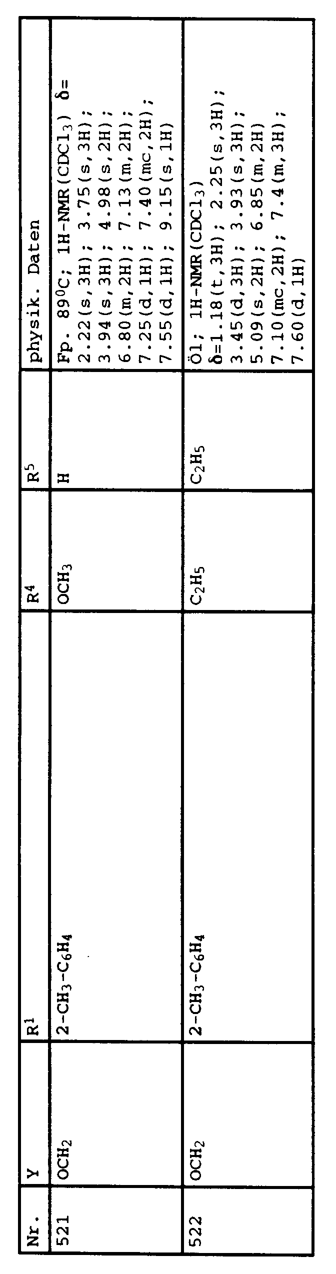

### Anwendungsbeispiele

Als Vergleichssubstanz diente 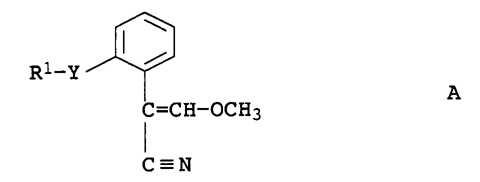 bekannt aus der EP-A 310 954 (Verbindung Nr. 312; E/Z-Isomerengemisch)

### Beispiel A

### Wirksamkeit gegen Rebenperonospora

Blätter von Topfreben der Sorte "Müller-Thurgau" wurden mit 0,025 Gew.-%-igen wäßrigen Suspensionen, die 80 Gew-% Wirkstoff (der Wirkstoffe gemäß den Tabellenbeispielen 83, 85, 89, 91, 225, 235, 411, 450, 451 und 522) und 20 Gew-% Emulgator in der Trockensubstanz enthielten, besprüht. Zur Beurteilung der Wirkungsdauer der Wirkstoffe wurden die Pflanzen nach dem Antrocknen des Spritzbelages 8 Tage im Gewächshaus aufgestellt. Erst dann wurden die Blätter mit einer Sporenaufschwemmung von Plasmopara viticola (Rebenperonospora) infiziert und die Pflanzen 48 Stunden in einer wasserdampfgesättigten Kammer bei 24°C aufgestellt. Anschließend wurden die Reben 5 Tage in einem Gewächshaus bei Temperaturen zwischen 20 und 30°C und zur Beschleunigung des Sporangienträgerausbruchs abermals für 16 Stunden in der feuchten Kammer kultiviert. Danach erfolgte die Beurteilung des Ausmaßes des Pilzbefalls auf den Blattunterseiten.

Gegenüber einem Kontrollversuch (keine Behandlung; 60 % Pilzbefall) und der bekannten Vergleichsverbindung A (35 % Pilzbefall) zeigte sich, daß die mit den Wirkstoffen 83, 85, 89, 91, 225, 235, 411, 450, 451 und 522 behandelten Pflanzen nur einen Pilzbefall von 0 bis 5 % hatten.

### Beispiel B

### Wirksamkeit gegen Weizenmehltaudie

Blätter von in Töpfen gewachsenen Weizenkeimlingen der Sorte "Frühgold" wurden mit 0,025 Gew.-%-igen wäßrigen Wirkstoffaufbereitungen, 80 Gew.-% Wirkstoff (der Wirkstoffe gemäß den Tabellenbeispielen 411 und 523) und 20 Gew.% Emulgator in der Trockensubstanz enthielten, besprüht und 24 Stunden nach dem Antrocknen des Spritzbelages mit Sporen des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung beurteilt.

Gegenüber einem Kontrollversuch (keine Behandlung; 70 % Pilzbefall) und der bekannten Vergleichsverbindung A (35 % Pilzbefall) zeigte sich, daß die mit den Wirkstoffen 411 und 523 behandelten Pflanzen kinen Pilzbefall aufwiesen.

### Beispiel C

### Wirksamkeit gegen Pyricularia oryzae (vorbeugende Behandlung)

Blätter von in Töpfen gewachsenen Reiskeimlingen der Sorte "Bahia" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae infiziert. Die Versuchspflanzen wurden anschließend in Klimakammern bei Temperaturen zwischen 20 und 24°C und 95-99 % relativer Luftfeuchtigkeit aufgestellt. Nach 6 Tagen wurde das Ausmaß des Pilzbefalls ermittelt.

Das Ergebnis zeigt, daß die Wirkstoffe 225, 235, 411, 521 und 524 bei der Anwendung als 0,05 %-ige (Gew.-%) wäßrige Wirkstoffaufbereitung eine viel bessere fungizide Wirkung aufweisen (93 %) als die bekannte Vergleichssubstanz A (20 %).

## Patentansprüche

1. Verwendung eines ortho-substituierten Phenylessigsäureamids der allgemeinen Formel I, 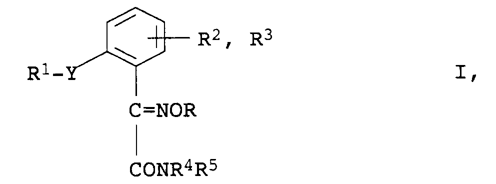 wobei die Variablen folgende Bedeutung haben:
R eine C₁-C₄-Alkylgruppe,
R¹ Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe
oder eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff-oder Schwefelatom anneliert sein kann und wobei der Heterocyclus zusätzlich noch ein Halogenatom, einen oder zwei C₁-C₄-Alkylreste oder einen Phenylrest tragen kann;
R², R³ unabhängig voneinander Wasserstoff, Cyano, Halogen, C₁-C₄-Alkyl oder C₁-C₄-Akoxy;
R⁴, R⁵ unabhängig voneinander Wasserstoff oder eine C₁-C₄-Alkylgruppe oder einer der beiden Substituenten eine C₁-C₄-Alkoxygruppe;
Y Sauerstoff oder Schwefel,
eine Gruppe SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette,
ausgenommen solche Verbindungen, in denen die Gruppe NR⁴R⁵ für NHCH₃ steht, Y Sauerstoff bedeutet, R² und R³ für Wasserstoff stehen, R für Methyl steht und R¹ Alkenyl bedeutet oder Phenyl bedeutet, welches
- einen der folgenden Reste trägt: Nitro, Halogen, Methyl, Isopropyl, tert.-Butyl, Methoxy, Phenoxy oder unsubstituiertes oder substituiertes Benzyloxy, oder welches
- zwei Chloratome oder zwei Isopropylgruppen trägt;
ausgenommen solche Verbindungen, in denen R² und R³ für Wasserstoff stehen, die Gruppe NR⁴R⁵ für NHCH₃ steht, Y OCH₂ bedeutet, R für Methyl steht und R¹ Phenyl bedeutet, welches ein Chloratom trägt; sowie
ausgenommen solche Verbindungen, in denen R² und R³ für Wasserstoff stehen, die Gruppe NR⁴R⁵ für NHCH₃ steht, Y OCH₂ bedeutet und R¹ für eine der folgenden Gruppen steht: 3-Phenoxyphenyl, 4-Cyanophenyl, 3-Nitrophenyl, 3-(N,N-Dimethylamino)-phenyl, 4-Fluorphenyl, 3-Bromphenyl, 4-Bromphenyl, 2,4-Dichlorphenyl, 3,4-Dichlorphenyl, 3,5-Dichlorphenyl, 4-Methylphenyl, 4-tert.-Butylphenyl, 3-Trifluormethylphenyl, 4-Methoxyphenyl, 3-Isopropyloxyphenyl, Pyrimidin-2-yl oder Pyridin-2-yl,
zur Bekämpfung von schädlingen, dadurch gekennzeichnet, daß man eine insektizid, nematizid und/oder akarizid wirksame Menge der Verbindungen I auf Insekten, Nematoden und/oder Akaridien bzw. deren Lebensraum einwirken läßt.

2. Ortho-substituierte Phenylessigsäureamide der allgemeinen Formel I.1, 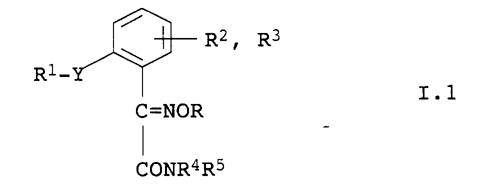 wobei die Variablen folgende Bedeutung haben:
R eine C₁-C₄-Alkylgruppe,
R¹ Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe
oder eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff-oder Schwefelatom anneliert sein kann und wobei der Heterocyclus zusätzlich noch ein Halogenatom, einen oder zwei C₁-C₄-Alkylreste oder einen Phenylrest tragen kann;
R², R³ unabhängig voneinander Wasserstoff, Cyano, Halogen, C₁-C₄-Alkyl oder C₁-C₄-Akoxy;
R⁴ Wasserstoff oder eine C₁-C₄-Alkylgruppe;
R⁵ eine C₁-C₄-Alkoxygruppe;
Y Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette.

3. Ortho-substitinierte Phenylessigsäureamide der allgemeinen Formel I.2, 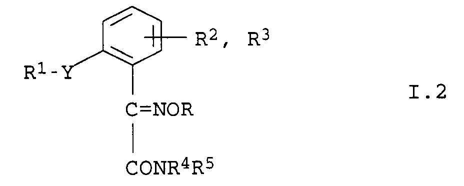 wobei die Variablen folgende Bedeutung haben:
R eine C₁-C₄-Alkylgruppe,
R¹ Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe
oder eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff-oder Schwefelatom anneliert sein kann und wobei der Heterocyclus zusätzlich noch ein Halogenatom, einen oder zwei C₁-C₄-Alkylreste oder einen Phenylrest tragen kann;
R² Wasserstoff, Cyano, Halogen, C₁-C₄-Alkyl oder C₁-C₄-Akoxy;
R³ Cyano;
R⁴, R⁵ unabhängig voneinander Wasserstoff oder eine C₁-C₄-Alkylgruppe oder einer der beiden Substituenten eine C₁-C₄-Alkoxygruppe;
Y Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette.

4. Ortho-substituierte Phenylessigsäureamide der allgemeinen Formel I.3, 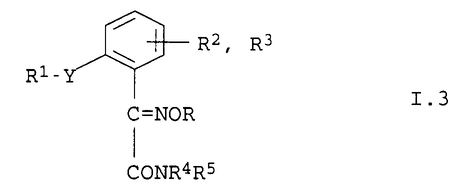 wobei die Variablen folgende Bedeutung haben:
R eine C₁-C₄-Alkylgruppe,
R¹ Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₁₀-Alkenylgruppe,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe
oder eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
die Phenylgruppe, welche noch einen bis fünf Substituenten trägt, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus mit einem bis drei Heteroatomen, ausgewählt aus einer Gruppe von zwei Sauerstoffatomen, zwei Schwefelatomen und drei Stickstoffatomen, ausgenommen Verbindungen mit zwei benachbarten Sauerstoff- und/oder Schwefelatomen, wobei an den Heterocyclus noch ein Benzolring oder ein 5- oder 6-gliedriger Heteroaromat mit einem Stickstoff-, Sauerstoff-oder Schwefelatom anneliert sein kann und wobei der Heterocyclus zusätzlich noch ein Halogenatom, einen oder zwei C₁-C₄-Alkylreste oder einen Phenylrest tragen kann;
R², R³ unabhängig voneinander Wasserstoff, Cyano, Halogen, C₁-C₄-Alkyl oder C₁-C₄-Akoxy;
R⁴, R⁵ unabhängig voneinander Wasserstoff oder eine C₁-C₄-Alkylgruppe oder einer der beiden Substituenten eine C₁-C₄-Alkoxygruppe;
Y eine Gruppe -SO₂-, -CH₂-O-SO₂-, -N=N-, -O-CO-, -CO-O- oder -CO-O-CH₂-,
eine C₁-C₄-Alkylenkette, die partiell oder vollständig halogeniert ist, oder die noch zusätzlich einen der folgenden Reste trägt: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine Carbonyl-C₁-C₄-alkylen- oder C₁-C₄-Alkylencarbonylkette.

5. Ortho-substituierte Phenylessigsäureamide der allgemeinen Formel I.4, 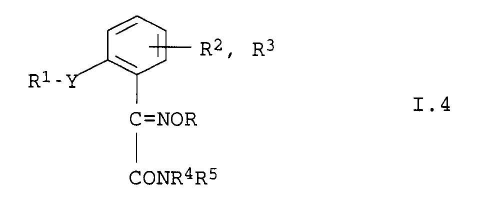 wobei die Variablen folgende Bedeutung haben:
R eine C₁-C₄-Alkylgruppe,
R¹ Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₁-C₄-Alkoxy-C₁-C₄-alkylgruppe, eine C₁-C₄-Alkoxycarbonylgruppe,
R², R³ unabhängig voneinander Wasserstoff, Cyano, Halogen, C₁-C₄-Alkyl oder C₁-C₄-Akoxy;
R⁴, R⁵ unabhängig voneinander Wasserstoff oder eine C₁-C₄-Alkylgruppe oder einer der beiden Substituenten eine C₁-C₄-Alkoxygruppe;
Y Sauerstoff oder Schwefel,
eine Gruppe -SO-, -SO₂-, -CH₂-O-SO₂-, -N=N-, -O-CO-, -CO-O- oder -CO-O-CH₂-,
eine C₁-C₄-Alkylenkette, die partiell oder vollständig halogeniert sein kann, und die noch zusätzlich einen der folgenden Reste tragen kann: Cyano, Nitro, C₁-C₄-Alkyl, partiell oder vollständig halogeniertes C₁-C₄-Alkyl, C₂-C₄-Alkenyl, partiell oder vollständig halogeniertes C₂-C₄-Alkenyl, C₁-C₄-Alkoxy, Phenyl oder Phenoxy, wobei die letzten beiden Reste ihrerseits noch einen oder zwei der folgenden Substituenten tragen können: Cyano, Halogen oder C₁-C₄-Alkyl,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette oder eine Carbonyl-C₁-C₄-alkylen- oder C₁-C₄-Alkylencarbonylkette.

6. Ortho-substituierte Phenylessigsäureamide der allgemeinen Formel I.5, 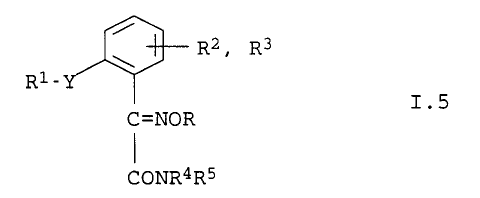 wobei die Variablen folgende Bedeutung haben:
R eine C₁-C₄-Alkylgruppe,
R¹ Wasserstoff, eine C₁-C₁₈-Alkylgruppe,
eine C₃-C₈-Cycloalkylgruppe die noch einen bis drei Substituenten tragen kann, ausgewählt aus einer Gruppe von 3 Halogenatomen, 3 C₁-C₄-Alkylgruppen, einer partiell oder vollständig halogenierten C₂-C₄-Alkenylgruppe und einer Phenylgruppe, die noch ein bis zwei Halogenatome und/oder eine C₁-C₄-Alkylgruppe und/oder eine C₁-C₄-Alkoxygruppe tragen kann,
eine C₂-C₄-Alkinylgruppe, die einen Phenylrest tragen kann,
eine C₁-C₄-Alkoxy-C₁-C₄-alkylgruppe, eine C₁-C₄-Alkoxycarbonylgruppe,
eine Phenyl-C₁-C₄-alkyl- oder Phenyl-C₂-C₄-alkenylgruppe
oder eine Phenoxy-C₁-C₄-alkylgruppe, wobei der Aromat jeweils noch einen bis fünf Substituenten tragen kann, ausgewählt aus einer Gruppe von 2 Nitroresten, 2 Cyanoresten, 5 Halogenatomen und jeweils drei der folgenden Reste: C₁-C₄-Alkyl, partiell oder vollständig halogeniertes C₁-C₄-Alkyl, C₂-C₄-Alkenyl, partiell oder vollständig halogeniertes C₂-C₄-Alkenyl und C₁-C₄-Alkoxy und einem Phenyl-, Benzyl-, Phenoxy-, Benzyloxy- oder Phenylthiorest, wobei die letzten beiden Reste ihrerseits noch einen oder zwei der folgenden Substituenten tragen können: Cyano, Halogen oder C₁-C₄-Alkyl;
einen 5- oder 6-gliedrigen Heterocyclus ausgewählt aus der Gruppe: Pyrrol-2-yl, Pyrrol-3-yl, Furan-2-yl, Furan-3-yl, Thien-2-yl, Thien-3-yl, Pyrazol-3-yl, Pyrazol-4-yl, Pyrazol-5-yl, Oxazol-2-yl, Oxazol-4-yl, Oxazol-5-yl, Benzoxazol-2-yl, Thiazol-2-yl, Thiazol-4-yl, Thiazol-5-yl, Benzothiazol-2-yl, Isoxazol-3-yl, Isoxazol-4-yl, Isoxazol-5-yl, Isothiazol-3-yl, Isothiazol-4-yl, Isothiazol-5-yl, Imidazol-2-yl, Imidazol-4-yl, 1,2,4-Oxadiazol-3-yl, 1,2,4-Oxadiazol-5-yl, 1,2,4-Thiadiazol-3-yl, 1,2,4-Thiadiazol-5-yl, 1,3,4-Oxadiazol-2-yl, 1,3,4-Thiadiazol-2-yl, 1,2,4-Triazol-3-yl, 1,3,4-Triazol-2-yl, Pyridin-3-yl, Pyridin-4-yl, Pyridazin-3-yl, Pyridazin-4-yl, Pyrimidin-4-yl, Pyrimidin-5-yl, Pyrazin-2-yl, 1,3,5-Triazin-2-yl und 1,2,4-Triazin-3-yl, wobei der Heterocyclus zusätzlich noch ein Halogenatom, einen oder zwei C₁-C₄-Alkylreste oder einen Phenylrest tragen kann;
R², R³ unabhängig voneinander Wasserstoff, Cyano, Halogen, C₁-C₄-Alkyl oder C₁-C₄-Akoxy;
R⁴, R⁵ unabhängig voneinander Wasserstoff oder eine C₁-C₄-Alkylgruppe oder einer der beiden Substituenten eine C₁-C₄-Alkoxygruppe;
Y Sauerstoff oder Schwefel, -SO-,
eine C₂-C₄-Alkenylen- oder C₂-C₄-Alkinylenkette, eine Oxy-C₁-C₄-alkylen-, Thio-C₁-C₄-alkylen- oder C₁-C₄-Alkylenoxykette.

7. Ortho-substituierte Phenylessigsäureamide der allgemeinen Formel I.6, 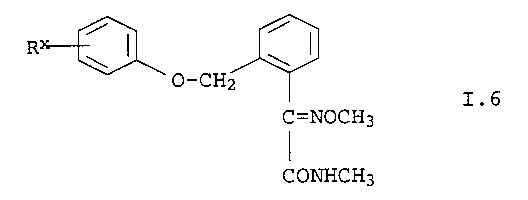 wobei R^{x} für die folgenden Substituenten bzw. Substituentenkombinationen steht:
2-Fluor, 3-Fluor, 2-Brom, 2-Iod, 2-Methyl, 3-Methyl, 2-Methoxy, 3-Methoxy, 2-Trifluormethyl, 4-Trifluormethyl, 2-Nitro, 4-Nitro, 2-Chlormethyl, 3-Chlormethyl, 4-Chlormethyl, 2-Ethyl, 3-Ethyl, 4-Ethyl, 3-iso-Propyl, 4-iso-Propyl, 3-tert.-Butyl, 3-Phenyl, 4-Phenyl, 3,5-Diethyl, 4-Cyclohexyl, 4-iso-Propyloxy, 4-tert.-Butyloxy, 4-Phenoxy, 3-Benzyloxy, 4-Benzyloxy, 2,3-Dichloro, 2,5-Dichloro, 2,6-Dichloro, 2,3,4-Trichloro, 2,3,5-Trichloro, 2,3,6-Trichloro, 3,4,5-Trichloro, Pentachloro, Pentafluoro, 2-Fluor-4-chlor, 4-Fluor-2-chlor, 2-Methyl-4-tert.-butyl, 2-Methyl-4-cyclohexyl, 2-Methyl-4-propyl, 2,3-Dimethyl, 2,4-Dimethyl, 2,5-Dimethyl, 3,4-Dimethyl, 3,5-Dimethyl, 2,3,5-Trimethyl oder 2,4,6-Trimethyl.

8. Ortho-substituierte Phenylessigsäureamide der allgemeinen Formel I.7, 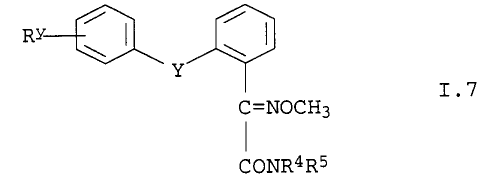 wobei die Variablen folgende Bedeutung haben:
R⁴, R⁵ unabhängig voneinander Wasserstoff oder Methyl;
Y CH₂CH₂, CH=CH oder CH₂O,
R^{y} 2-Fluor, 3-Fluor, 4-Fluor, 2-Chlor, 3-Chlor, 4-Chlor, 2-Brom, 4-Brom, 2-Jod, 2-Methyl, 3-Methyl, 4-Methyl, 2-Methoxy, 3-Methoxy, 4-Methoxy, 2-Trifluormethyl, 3-Trifluormethyl, 4-Trifluormethyl, 2,4-Dichlor, 2,4-Dimethyl oder 2,4,6-Trimethyl.

9. Verfahren zur Herstellung der Verbindungen I.1 bis I.7 gemäß den Ansprüchen 2 bis 8, dadurch gekennzeichnet, daß man ein Phenylessigsäurederivat der Formel II 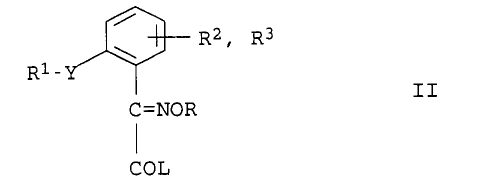 wobei L Halogen oder C₁-C₄-Alkoxy bedeutet, gewünschtenfalls in Gegenwart einer Base mit einem Amin der Formel III
HNR⁴R⁵ III
umsetzt.

10. Verfahren zur Herstellung der Verbindungen I.1 bis I.7 gemäß den Ansprüchen 2 bis 8, dadurch gekennzeichnet, daß man ein Phenylacetonitril der Formel IV 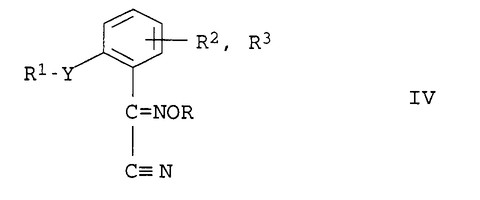 in Gegenwart einer Säure oder Base hydrolysiert und das Verfahrensprodukt am Amidstickstoff einmal alkyliert.

11. Phenylacetonitrile der Formel IV gemäß Anspruch 10.

12. Verwendung der Verbindungen IV gemäß Anspruch 11 als Zwischenprodukt.

13. Fungizides Mittel, enthaltend einen flüssigen oder festen Trägerstoff und mindestens eine Verbindung der Formel I.1 bis I.7 gemäß den Ansprüchen 2 bis 8.

14. Schädlingsbekämpfungsmittel, enthaltend einen inerten Trägerstoff und mindestens eine Verbindung der Formel I.1 bis I.7 gemäß den Ansprüchen 2 bis 8.

15. Verfahren zur Bekämpfung von Pilzen, dadurch gekennzeichnet, daß man eine fungizid wirksame Menge einer Verbindung der Formel I.1 bis I.7 gemäß den Ansprüchen 2 bis 8 auf Pilze, von Pilzbefall bedrohte Pflanzen, deren Lebensraum oder auf das Saatgut der bedrohten Pflanzen einwirken läßt.

## Claims

1. The use of an ortho-substituted phenylacetamide of the formula I 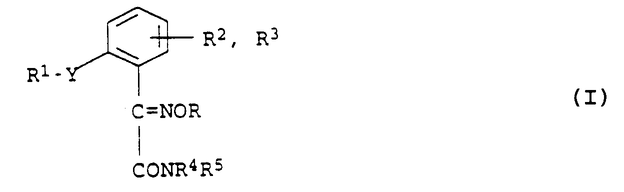 where
R is C₁-C₄-alkyl,
R¹ is hydrogen, C₁-C₁₈-alkyl,
C₃-C₈-cycloalkyl which can have from one to three substituents selected from a group of 3 halogen atoms, 3 C₁-C₄-alkyl groups, a partially or completely halogenated C₂-C₄-alkenyl group and a phenyl group which can carry one or two halogen atoms and/or one C₁-C₄-alkyl group and/or one C₁-C₄-alkoxy group,
C₂-C₁₀-alkenyl,
C₂-C₄-alkynyl which can carry a phenyl radical,
C₁-C₄-alkoxy-C₁-C₄-alkyl, C₁-C₄-alkoxycarbonyl,
phenyl-C₁-C₄-alkyl or phenyl-C₂-C₄-alkenyl or
phenoxy-C₁-C₄-alkyl where each aromatic ring can have from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
phenyl which has from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
a 5- or 6-membered heterocycle with from one to three hetero atoms selected from a group of two oxygen, two sulfur and three nitrogen atoms, excepting compounds with two adjacent oxygen and/or sulfur atoms, it being possible for a benzene ring or a 5- or 6-membered heteroaromatic ring with one nitrogen, oxygen or sulfur atom to be fused onto the heterocycle, and it being possible for the heterocycle to carry in addition one halogen atom, one or two C₁-C₄-alkyl radicals or one phenyl radical;
R² and R³ are each, independently of one another, hydrogen, cyano, halogen, C₁-C₄-alkyl or C₁-C₄-alkoxy;
R⁴ and R⁵ are each, independently of one another, hydrogen or C₁-C₄-alkyl or one of the two is C₁-C₄-alkoxy;
Y is oxygen or sulfur,
-SO-, -SO₂-, -CH₂-O-SO₂-, -N=N-, -O-CO-, -CO-O- or -CO-O-CH₂-,
C₁-C₄-alkylene which can be partially or completely halogenated and can carry in addition one of the following: cyano, nitro, C₁-C₄-alkyl, partially or completely halogenated C₁-C₄-alkyl, C₂-C₄-alkenyl, partially or completely halogenated C₂-C₄-alkenyl, C₁-C₄-alkoxy, phenyl or phenoxy, it being possible for the last two radicals in turn to have one or two of the following substituents: cyano, halogen or C₁-C₄-alkyl,
C₂-C₄-alkenylene or C₂-C₄-alkynylene, oxy-C₁-C₄-alkylene, thio-C₁-C₄-alkylene or C₁-C₄-alkyleneoxy or carbonyl-C₁-C₄-alkylene or C₁-C₄-alkylenecarbonyl,
excepting compounds where NR⁴R⁵ is NHCH₃, Y is oxygen, R² and R³ are hydrogen, R is methyl and R¹ is alkenyl or is phenyl which
- carries one of the following: nitro, halogen, methyl, isopropyl, tert-butyl, methoxy, phenoxy or unsubstituted or substituted benzyloxy, or which
- carries two chlorine atoms or two isopropyl groups;
excepting compounds where R² and R³ are hydrogen, NR⁴R⁵ is NHCH₃, Y is OCH₂, R is methyl and R¹ is phenyl which carries a chlorine atom; and
excepting compounds where R² and R³ are hydrogen, NR⁴R⁵ is NHCH₃, Y is OCH₂ and R¹ is one of the following: 3-phenoxyphenyl, 4-cyanophenyl, 3-nitrophenyl, 3-(N,N-dimethylamino)phenyl, 4-fluorophenyl, 3-bromophenyl, 4-bromophenyl, 2,4-dichlorophenyl, 3,4-dichlorophenyl, 3,5-dichlorophenyl, 4-methylphenyl, 4-tert-butylphenyl, 3-trifluoromethylphenyl, 4-methoxyphenyl, 3-isopropyloxyphenyl, 2-pyrimidinyl or 2-pyridinyl, for controlling pests, wherein insects, nematodes and/or acarids or their habitat are exposed to an insecticidal, nematicidal and/or acaricidal amount of the compound.

2. An ortho-substituted phenylacetamide of the formula I.1, 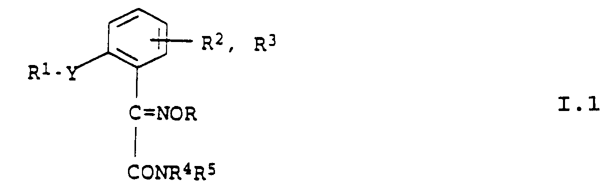 where
R is C₁-C₄-alkyl,
R¹ is hydrogen, C₁-C₁₈-alkyl,
C₃-C₈-cycloalkyl which can have from one to three substituents selected from a group of 3 halogen atoms, 3 C₁-C₄-alkyl groups, a partially or completely halogenated C₂-C₄-alkenyl group and a phenyl group which can carry one or two halogen atoms and/or one C₁-C₄-alkyl group and/or one C₁-C₄-alkoxy group,
C₂-C₁₀-alkenyl,
C₂-C₄-alkynyl which can carry a phenyl radical,
C₁-C₄-alkoxy-C₁-C₄-alkyl, C₁-C₄-alkoxycarbonyl,
phenyl-C₁-C₄-alkyl or phenyl-C₂-C₄-alkenyl or phenoxy-C₁-C₄-alkyl where each aromatic ring can have from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
phenyl which has from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
a 5- or 6-membered heterocycle with from one to three hetero atoms selected from a group of two oxygen, two sulfur and three nitrogen atoms, excepting compounds with two adjacent oxygen and/or sulfur atoms, it being possible for a benzene ring or a 5- or 6-membered heteroaromatic ring with one nitrogen, oxygen or sulfur atom to be fused onto the heterocycle, and it being possible for the heterocycle to carry in addition one halogen atom, one or two C₁-C₄-alkyl radicals or one phenyl radical;
R² and R³ are each, independently of one another, hydrogen, cyano, halogen, C₁-C₄-alkyl or C₁-C₄-alkoxy;
R⁴ is hydrogen or C₁-C₄-alkyl;
R⁵ is C₁-C₄-alkoxy;
Y is oxygen or sulfur,
-SO-, -SO₂-, -CH₂-O-SO₂-, -N=N-, -O-CO-, -CO-O- or -CO-O-CH₂-,
C₁-C₄-alkylene which can be partially or completely halogenated and can carry in addition one of the following: cyano, nitro, C₁-C₄-alkyl, partially or completely halogenated C₁-C₄-alkyl, C₂-C₄-alkenyl, partially or completely halogenated C₂-C₄-alkenyl, C₁-C₄-alkoxy, phenyl or phenoxy, it being possible for the last two radicals in turn to have one or two of the following substituents: cyano, halogen or C₁-C₄-alkyl,
C₂-C₄-alkenylene or C₂-C₄-alkynylene, oxy-C₁-C₄-alkylene, thio-C₁-C₄-alkylene or C₁-C₄-alkyleneoxy or carbonyl-C₁-C₄-alkylene or C₁-C₄-alkylenecarbonyl.

3. An ortho-substituted phenylacetamide of the formula I.2 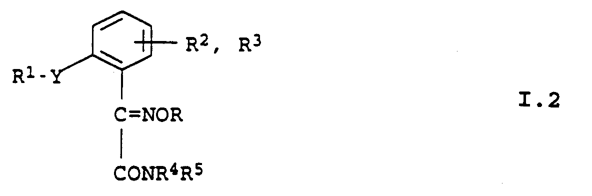 where
R is C₁-C₄-alkyl,
R¹ is hydrogen, C₁-C₁₈-alkyl,
C₃-C₈-cycloalkyl which can have from one to three substituents selected from a group of 3 halogen atoms, 3 C₁-C₄-alkyl groups, a partially or completely halogenated C₂-C₄-alkenyl group and a phenyl group which can carry one or two halogen atoms and/or one C₁-C₄-alkyl group and/or one C₁-C₄-alkoxy group,
C₂-C₁₀-alkenyl,
C₂-C₄-alkynyl which can carry a phenyl radical,
C₁-C₄-alkoxy-C₁-C₄-alkyl, C₁-C₄-alkoxycarbonyl,
phenyl-C₁-C₄-alkyl or phenyl-C₂-C₄-alkenyl or phenoxy-C₁-C₄-alkyl where each aromatic ring can have from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
phenyl which has from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
a 5- or 6-membered heterocycle with from one to three hetero atoms selected from a group of two oxygen, two sulfur and three nitrogen atoms, excepting compounds with two adjacent oxygen and/or sulfur atoms, it being possible for a benzene ring or a 5- or 6-membered heteroaromatic ring with one nitrogen, oxygen or sulfur atom to be fused onto the heterocycle, and it being possible for the heterocycle to carry in addition one halogen atom, one or two C₁-C₄-alkyl radicals or one phenyl radical;
R² is hydrogen, cyano, halogen, C₁-C₄-alkyl or C₁-C₄-alkoxy;
R³ is cyano;
R⁴ and R⁵ are each, independently of one another, hydrogen or C₁-C₄-alkyl or one of the two is C₁-C₄-alkoxy;
Y is oxygen or sulfur,
-SO-, -SO₂-, -CH₂-O-SO₂-, -N=N-, -O-CO-, -CO-O- or -CO-O-CH₂-,
C₁-C₄-alkylene which can be partially or completely halogenated and can carry in addition one of the following: cyano, nitro, C₁-C₄-alkyl, partially or completely halogenated C₁-C₄-alkyl, C₂-C₄-alkenyl, partially or completely halogenated C₂-C₄-alkenyl, C₁-C₄-alkoxy, phenyl or phenoxy, it being possible for the last two radicals in turn to have one or two of the following substituents: cyano, halogen or C₁-C₄-alkyl,
C₂-C₄-alkenylene or C₂-C₄-alkynylene, oxy-C₁-C₄-alkylene, thio-C₁-C₄-alkylene or C₁-C₄-alkyleneoxy or carbonyl -C₁-C₄-alkylene or C₁-C₄-alkylenecarbonyl.

4. An ortho-substituted phenylacetamide of the formula I.3 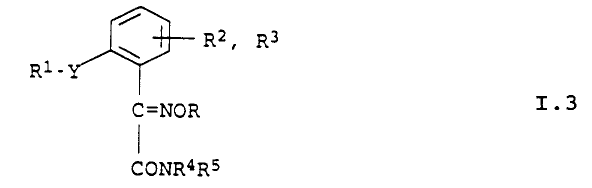 where
R is C₁-C₄-alkyl,
R¹ is hydrogen, C₁-C₁₈-alkyl,
C₃-C₈-cycloalkyl which can have from one to three substituents selected from a group of 3 halogen atoms, 3 C₁-C₄-alkyl groups, a partially or completely halogenated C₂-C₄-alkenyl group and a phenyl group which can carry one or two halogen atoms and/or one C₁-C₄-alkyl group and/or one C₁-C₄-alkoxy group,
C₂-C₁₀-alkenyl,
C₂-C₄-alkynyl which can carry a phenyl radical,
C₁-C₄-alkoxy-C₁-C₄-alkyl, C₁-C₄-alkoxycarbonyl,
phenyl-C₁-C₄-alkyl or phenyl-C₂-C₄-alkenyl or phenoxy-C₁-C₄-alkyl where each aromatic ring can have from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
phenyl which has from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
a 5- or 6-membered heterocycle with from one to three hetero atoms selected from a group of two oxygen, two sulfur and three nitrogen atoms, excepting compounds with two adjacent oxygen and/or sulfur atoms, it being possible for a benzene ring or a 5- or 6-membered heteroaromatic ring with one nitrogen, oxygen or sulfur atom to be fused onto the heterocycle, and it being possible for the heterocycle to carry in addition one halogen atom, one or two C₁-C₄-alkyl radicals or one phenyl radical;
R² and R³ are each, independently of one another, hydrogen, cyano, halogen, C₁-C₄-alkyl or C₁-C₄-alkoxy;
R⁴ and R⁵ are each, independently of one another, hydrogen or C₁-C₄-alkyl or one of the two is C₁-C₄-alkoxy;
Y is -SO₂-, -CH₂-O-SO₂-, -N=N-, -O-CO-, -CO-O- or -CO-O-CH₂-,
C₁-C₄-alkylene which is partially or completely halogenated or carries in addition one of the following: cyano, nitro, C₁-C₄-alkyl, partially or completely halogenated C₁-C₄-alkyl, C₂-C₄-alkenyl, partially or completely halogenated C₂-C₄-alkenyl, C₁-C₄-alkoxy, phenyl or phenoxy, it being possible for the last two radicals in turn to have one or two of the following substituents: cyano, halogen or C₁-C₄-alkyl,
or carbonyl -C₁-C₄-alkylene or C₁-C₄-alkylenecarbonyl.

5. An ortho-substituted phenylacetamide of the formula I.4 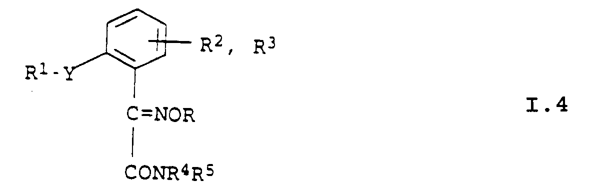 where
R is C₁-C₄-alkyl,
R¹ is hydrogen, C₁-C₁₈-alkyl,
C₃-C₈-cycloalkyl which can have from one to three substituents selected from a group of 3 halogen atoms, 3 C₁-C₄-alkyl groups, a partially or completely halogenated C₂-C₄-alkenyl group and a phenyl group which can carry one or two halogen atoms and/or one C₁-C₄-alkyl group and/or one C₁-C₄-alkoxy group,
C₁-C₄-alkoxy-C₁-C₄-alkyl, C₁-C₄-alkoxycarbonyl,
R² and R³ are each, independently of one another, hydrogen, cyano, halogen, C₁-C₄-alkyl or C₁-C₄-alkoxy;
R⁴ and R⁵ are each, independently of one another, hydrogen or C₁-C₄-alkyl or one of the two is C₁-C₄-alkoxy;
Y is oxygen or sulfur,
-SO-, -SO₂-, -CH₂-O-SO₂-, -N=N-, -O-CO-, -CO-O- or -CO-O-CH₂-,
C₁-C₄-alkylene which can be partially or completely halogenated and can carry in addition one of the following: cyano, nitro, C₁-C₄-alkyl, partially or completely halogenated C₁-C₄-alkyl, C₂-C₄-alkenyl, partially or completely halogenated C₂-C₄-alkenyl, C₁-C₄-alkoxy, phenyl or phenoxy, it being possible for the last two radicals in turn to have one or two of the following substituents: cyano, halogen or C₁-C₄-alkyl,
C₂-C₄-alkenylene or C₂-C₄-alkynylene, oxy-C₁-C₄-alkylene, thio-C₁-C₄-alkylene or C₁-C₄-alkyleneoxy or carbonyl-C₁-C₄-alkylene or C₁-C₄-alkylenecarbonyl.

6. An ortho-substituted phenylacetamide of the formula I.5 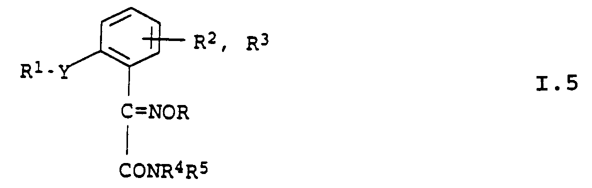 where
R is C₁-C₄-alkyl,
R¹ is hydrogen, C₁-C₁₈-alkyl,
C₃-C₈-cycloalkyl which can have from one to three substituents selected from a group of 3 halogen atoms, 3 C₁-C₄-alkyl groups, a partially or completely halogenated C₂-C₄-alkenyl group and a phenyl group which can carry one or two halogen atoms and/or one C₁-C₄-alkyl group and/or one C₁-C₄-alkoxy group,
C₂-C₄-alkynyl which can carry a phenyl radical,
C₁-C₄-alkoxy-C₁-C₄-alkyl, C₁-C₄-alkoxycarbonyl,
phenyl-C₁-C₄-alkyl or phenyl-C₂-C₄-alkenyl or phenoxy-C₁-C₄-alkyl where each aromatic ring can have from one to five substituents selected from a group of 2 nitro radicals, 2 cyano radicals, 5 halogen atoms and in each case three of the following: C₁-C₄-alkyl, partially or completely halogenated C₁-C₄-alkyl, C₂-C₄-alkenyl, partially or completely halogenated C₂-C₄-alkenyl and C₁-C₄-alkoxy, and of one phenyl, benzyl, phenoxy, benzyloxy or phenylthio radical, where the last two radicals in turn can have one or two of the following substituents: cyano, halogen or C₁-C₄-alkyl;
a 5- or 6-membered heterocycle selected from the group: 2-pyrrolyl, 3-pyrrolyl, 2-furanyl, 3-furanyl, 2-thienyl, 3-thienyl, 3-pyrazolyl, 4-pyrazolyl, 5-pyrazolyl, 2-oxazolyl, 4-oxazolyl, 5-oxazolyl, 2-benzoxazolyl, 2-thiazolyl, 4-thiazolyl, 5-thiazolyl, 2-benzothiazolyl, 3-isoxazolyl, 4-isoxazolyl, 5-isoxazolyl, 3-isothiazolyl, 4-isothiazolyl, 5-isothiazolyl, 2-imidazolyl, 4-imidazolyl, 1,2,4-oxadiazol-3-yl, 1,2,4-oxadiazol-5-yl, 1,2,4-thiadiazol-3-yl, 1,2,4-thiadiazol-5-yl, 1,3,4-oxadiazol-2-yl, 1,3,4-thiadiazol-2-yl, 1,2,4-triazol-3-yl, 1,3,4-triazol-2-yl, 3-pyridinyl, 4-pyridinyl, 3-pyridazinyl, 4-pyridazinyl, 4-pyrimidinyl, 5-pyrimidinyl, 2-pyrazinyl, 1,3,5-triazin-2-yl and 1,2,4-triazin-3-yl, it also being possible for the heterocycle to carry in addition a halogen atom, one or two C₁-C₄-alkyl radicals or a phenyl radical;
R² and R³ are each, independently of one another, hydrogen, cyano, halogen, C₁-C₄-alkyl or C₁-C₄-alkoxy;
R⁴ and R⁵ are each, independently of one another, hydrogen or C₁-C₄-alkyl, or one of the two is C₁-C₄-alkoxy;
Y is oxygen or sulfur, -SO-,
C₂-C₄-alkenylene or C₂-C₄-alkynylene, oxy-C₁-C₄-alkylene, thio-C₁-C₄-alkylene or C₁-C₄-alkyleneoxy.

7. An ortho-substituted phenylacetamide of the formula I.6, 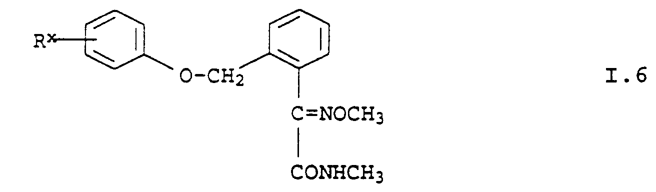 where R^{x} represents the following substituents or combinations of substituents:
2-fluoro, 3-fluoro, 2-bromo, 2-iodo, 2-methyl, 3-methyl, 2-methoxy, 3-methoxy, 2-trifluoromethyl, 4-trifluoromethyl, 2-nitro, 4-nitro, 2-chloromethyl, 3-chloromethyl, 4-chloromethyl, 2-ethyl, 3-ethyl, 4-ethyl, 3-isopropyl, 4-isopropyl, 3-tert-butyl, 3-phenyl, 4-phenyl, 3,5-diethyl, 4-cyclohexyl, 4-iso-propyloxy, 4-tert-butyloxy, 4-phenoxy, 3-benzyloxy, 4-benzyloxy, 2,3-dichloro, 2,5-dichloro, 2,6-dichloro, 2,3,4-trichloro, 2,3,5-trichloro, 2,3,6-trichloro, 3,4,5-trichloro, pentachloro, pentafluoro, 2-fluoro-4-chloro, 4-fluoro-2-chloro, 2-methyl-4-tert-butyl, 2-methyl-4-cyclohexyl, 2-methyl-4-propyl, 2,3-dimethyl, 2,4-dimethyl, 2,5-dimethyl, 3,4-dimethyl, 3,5-dimethyl, 2,3,5-trimethyl or 2,4,6-trimethyl.

8. An ortho-substituted phenylacetamide of the formula I.7, 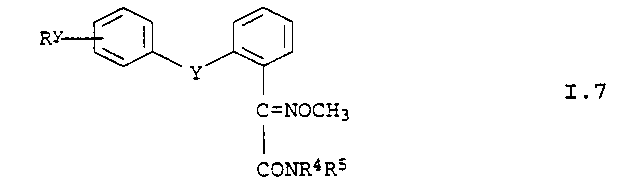 where
R⁴ and R⁵ are each, independently of one another, hydrogen or methyl;
Y is CH₂CH₂, CH=CH or CH₂O,
R^{y} is 2-fluoro, 3-fluoro, 4-fluoro, 2-chloro, 3-chloro, 4-chloro, 2-bromo, 4-bromo, 2-iodo, 2-methyl, 3-methyl, 4-methyl, 2-methoxy, 3-methoxy, 4-methoxy, 2-trifluoromethyl, 3-trifluoromethyl, 4-trifluoromethyl, 2,4-dichloro, 2,4-dimethyl or 2,4,6-trimethyl.

9. A process for preparing the compounds I.1 to I.7 as claimed in claims 2 to 8, which comprises reacting a phenylacetic acid derivative of the formula II 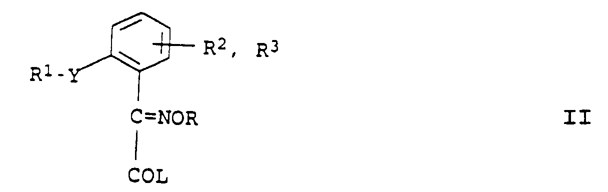 where L is halogen or C₁-C₄-alkoxy, if desired in the presence of a base, with an amine of the formula III
HNR⁴R⁵ III

10. A process for preparing the compounds I.1 to I.7 as claimed in claims 2 to 8, which comprises hydrolyzing a phenylacetonitrile of the formula IV 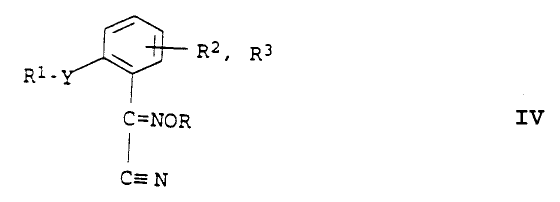 in the presence of an acid or base and alkylating the product once on the amide nitrogen.

11. A phenylacetonitrile of the formula IV as set-forth in claim 10.

12. The use of a compound IV as claimed in claim 11 as an intermediate.

13. A fungicidal agent containing a liquid or solid carrier and at least one compound of the formula I.1 to I.7 as claimed in claims 2 to 8.

14. A pesticide containing an inert carrier and at least one compound of the formula I.1 to I.7 as claimed in claims 2 to 8.

15. A method for controlling fungi, which comprises exposing the fungi, the plants threatened by fungal attack, their habitat or the seed of the threatened plants to a fungicidal amount of a compound of the formula I.1 to I.7 as claimed in claims 2 to 8.

## Revendications

1. Utilisation d'un phénylacétamide substitue en ortho et répondant à la formule générale I 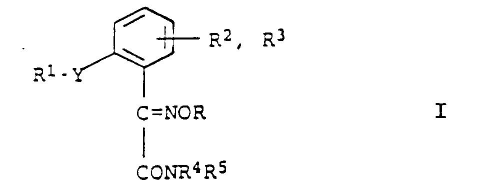 dans laquelle les symboles ont les significations suivantes :
R : un groupe alkyle en C1-C4,
R¹ : l'hydrogène, un groupe alkyle en C1-C8,
un groupe cycloalkyle en C3-C8 qui peut encore porter un à trois substituants choisis dans un groupe consistant en trois atomes d'halogènes, trois groupes alkyle en C1-C4, un groupe alcényle en C2-C4 partiellement ou totalement halogéné et un groupe phényle qui peut encore porter un à deux atomes d'halogènes et/ou un groupe alkyle en C1-C4 et/ou un groupe alcoxy en C1-C4,
un groupe alcényle en C2-C10,
un groupe alcynyle en C2-C4, qui peut porter un groupe phényle,
un groupe (alcoxy en C1-C4)alkyle en C1-C4,
un groupe (alcoxy en C1-C4)carbonyle,
un groupe phényl-alkyle en C1-C4 ou phényl-alcényle en C2-C4 ou un groupe phénoxy-alkyle en C1-C4, la partie aromatique pouvant dans chaque cas porter encore un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4, et un groupe phényle, benzyle, phénoxy, benzyloxy ou phénylthio, ces deux derniers groupes pouvant eux-mêmes porter encore un ou deux des substituants suivants : cyano, halogéno ou alkyle en C1-C4 ;
le groupe phényle qui peut encore porter un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4, et un groupe phényle, benzyle, phénoxy, benzyloxy ou phénylthio, ces deux derniers pouvant eux-mêmes porter encore un ou deux des substituants suivants : cyano, halogéno ou alkyle en C1-C4 ;
un hétérocycle à cinq ou six chaînons contenant un à trois hétéroatomes choisis dans un groupe consistant en deux atomes d'oxygène, deux atomes de soufre et trois d'atomes d'azote, à l'exception des composés qui contiennent deux atomes d'oxygène et/ou de soufre voisins, l'hétérocycle pouvant encore être condensé sur un noyau benzénique ou sur un cycle hétéroaromatique à cinq ou six chaînons portant un atome d'azote, d'oxygène ou de soufre, l'hétérocycle pouvant encore porter un atome d'halogène, un ou deux groupes alkyle en C1-C4 ou un groupe phényle ;
R² et R³ représentent chacun, indépendamment l'un de l'autre, l'hydrogène, un groupe cyano, un halogène, un groupe alkyle en C1-C4 ou alcoxy en C1-C4 ;
R⁴ et R⁵ représentent chacun, indépendamment l'un de l'autre, l'hydrogène ou un groupe alkyle en C1-C4, l'un de ces deux symboles pouvant encore représenter un groupe alcoxy en C1-C4 ;
Y représente l'oxygène ou le soufre ;
un groupe -SO-, -SO₂-, -CH₂-O-SO₂-, -N=N-, -O-CO-, - CO-O- ou -CO-O-CH₂,
une chaîne alkylène en C1-C4 qui peut être partiellement ou totalement halogénée et qui peut en outre porter l'un des substituants suivants : cyano, nitro, alkyle en C1-C4, alkyle en C1-C4 partiellement ou totalement halogéné, alcényle en C2-C4, alcényle en C2-C4 partiellement ou totalement halogéné, alcoxy en C1-C4, phényle ou phénoxy, ces deux derniers groupes pouvant eux-mêmes porter un ou deux des substituants suivants : cyano, halogéno ou alkyle en C1-C4,
une chaîne alcénylène en C2-C4 ou alcynylène en C2-C4, une chaîne oxy-alkylène en C1-C4, thio-alkylène en C1-C4 ou alkylène-oxy en C1-C4 ou une chaîne carbonyl-alkylène en C1-C4 ou alkylène en C1-C4-carbonyle,
à l'exception des composés pour lesquels le groupe NR⁴R⁵ consiste en NHCH₃, Y représente l'oxygène, R² et R³ représentent l'hydrogène, R un groupe méthyle et R¹ un groupe alcényle ou phényle, lequel porte
- l'un des substituants suivants : nitro, halogéno, méthyle, isopropyle, tert-butyle, méthoxy, phénoxy ou benzyloxy éventuellement substitué,
ou qui porte
- deux atomes de chlore ou deux groupes isopropyle ;
à l'exception des composés pour lesquels R² et R³ représentant l'hydrogène, le groupe NR⁴R⁵ consiste en NHCH₃, Y représente OCH₂, R un groupe méthyle et R¹ un groupe phényle qui porte un atome de chlore ; et également
à l'exception des composés pour lesquels R² et R³ représentent l'hydrogène, le groupe NR⁴R⁵ consiste en NHCH₃, Y représente OCH₂ et R¹ représente l'un des groupes suivants : 3-phénoxyphényle, 4-cyanophényle, 3-nitrophényle, 3-(N,N-diméthylamino)phényle, 4-fluorophényle, 3-bromophényle, 4-bromophényle, 2,4-dichlorophényle, 3,4-dichlorophényle, 3,5-dichlorophényle, 4-méthylphényle, 4-tert-butylphényle, 3-trifluorométhylphényle, 4-méthoxyphényle, 3-isopropyloxyphényle, pyrimidin-2-yle ou pyridin-2-yle,
pour la lutte contre les parasites, caractérisé par le fait que l'on fait agir une quantité insecticide, nématocide et/ou acaricide efficace de ces composés sur les insectes, les nématodes et/ou les acariens ou leur habitat.

2. Phénylacétamides substitués en ortho, répondant à la formule générale I.1 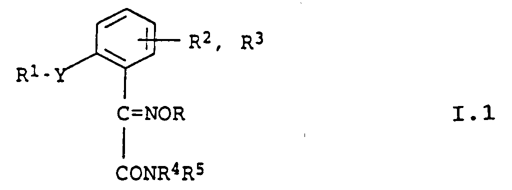 dans laquelle les symboles ont les significations suivantes :
R : un groupe alkyle en C1-C4,
R¹ : l'hydrogène, un groupe alkyle en C1-C18,
un groupe cycloalkyle en C3-C8 qui peut encore porter un à trois substituants choisis dans un groupe consistant en trois atomes d'halogènes, trois groupes alkyle en C1-C4, un groupe alcényle en C2-C4 partiellement ou totalement halogéné et un groupe phényle qui peut lui-même porter encore un à deux atomes d'halogènes et/ou un groupe alkyle en C1-C4 et/ou un groupe alcoxy en C1-C4,
un groupe alcényle en C2-C10,
un groupe alcynyle en C2-C4 qui peut porter un groupe phényle,
un groupe (alcoxy en C1-C4)alkyle en C1-C4, un groupe (alcoxy en C1-C4)carbonyle,
un groupe phényl-alkyle en C1-C4 ou phényl-alcényle en C2-C4 ou un groupe phénoxy-alkyle en C1-C4, dans lesquels la partie aromatique peut encore porter un à cinq substituants choisis dans un groupe consistant en deux groupes nitro, deux groupes cyano, cinq atomes d'halogènes et trois de chacun des substituants suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers groupes pouvant eux-mêmes porter un ou deux des substituants suivants : cyano, halogéno ou alkyle en C1-C4 ;
le groupe phényle, qui peut encore porter un à cinq substituants choisis dans un groupe consistant en deux groupes nitro, deux groupes cyano, cinq atomes d'halogènes et trois de chacun des substituants suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers groupes pouvant eux-mêmes porter un ou deux des substituants suivants : cyano, halogéno ou alkyle en C1-C4 ;
un hétérocycle à cinq ou six chaînons contenant un à trois hétéroatomes choisis dans un groupe consistant en deux atomes d'oxygène, deux atomes de soufre et trois atomes d'azote, à l'exception des composés qui contiennent deux atomes d'oxygène et/ou de soufre voisins, l'hétérocycle pouvant encore être condensé sur un cycle benzénique ou sur un cycle hétéroaromatique à cinq ou six chaînons contenant un atome d'azote, d'oxygène ou de soufre, l'hétérocycle pouvant encore porter un atome d'halogène, un ou deux groupes alkyle en C1-C4 ou un groupe phényle ;
R² et R³ représentent chacun, indépendamment l'un de l'autre, l'hydrogène, un groupe cyano, un halogène, un groupe alkyle en C1-C4 ou alcoxy en C1-C4 ;
R⁴ : l'hydrogène ou un groupe alkyle en C1-C4 ;
R⁵ : un groupe alcoxy en C1-C4 ;
Y : l'oxygène ou le soufre,
un groupe -SO-, -SO₂-, -CH₂-O-SO₂-, -N=N-, -O-CO-, - CO-O- ou -CO-O-CH₂,
une chaîne alkylène en C1-C4 qui peut être partiellement ou totalement halogénée et qui peut en outre porter l'un des substituants suivants : cyano, nitro, alkyle en C1-C4, alkyle en C1-C4 partiellement ou totalement halogéné, alcényle en C2-C4, alcényle en C2-C4 partiellement ou totalement halogéné, alcoxy en C1-C4, phényle ou phénoxy, ces deux derniers groupes pouvant eux-mêmes porter un ou deux des substituants suivants : cyano, halogéno ou alkyle en C1-C4,
une chaîne alcénylène en C2-C4 ou alcynylène en C2-C4, une chaîne oxy-alkylène en C1-C4, thio-alkylène en C1-C4 ou alkylène-oxy en C1-C4 ou carbonyl-alkylène en C1-C4 ou alkylène en C1-C4-carbonyle.

3. Phénylacétamides substitués en ortho, répondant à la formule générale I.2, 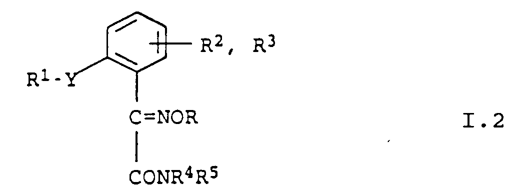 dans laquelle les symboles ont les significations suivantes :
R : un groupe alkyle en C1-C4,
R¹ : l'hydrogène, un groupe alkyle en C1-C18,
un groupe cycloalkyle en C3-C8 qui peut encore porter un à trois substituants choisis dans un groupe consistant en trois atomes d'halogènes, trois groupes alkyle en C1-C4, un groupe alcényle en C2-C4 partiellement ou totalement halogéné et un groupe phényle, lequel peint encore porter un à deux atomes d'halogènes et/ou un groupe alkyle en C1-C4 et/ou un groupe alcoxy en C1-C4,
un groupe alcényle en C2-C10,
un groupe alcynyle en C2-C4 qui peut porter un groupe phényle,
un groupe (alcoxy en C1-C4)alkyle en C1-C4, un groupe (alcoxy en C1-C4)carbonyle,
un groupe phényl-alkyle en C1-C4 ou phényl-alcényle en C2-C4 ou phénoxy-alkyle en C1-C4, chacune des parties aromatiques pouvant encore porter un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers groupes pouvant eux-mêmes porter un ou deux des substituants suivants : cyano, halogéno ou alkyle en C1-C4 ;
le groupe phényle, qui peut encore porter un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers groupes pouvant eux-mêmes porter un ou deux des substituants suivants : cyano, halogéno ou alkyle en C1-C4 ;
un hétérocycle à cinq ou six chaînons contenant trois hétéroatomes choisis dans un groupe consistant en deux atomes d'oxygène, deux atomes de soufre et trois atomes d'azote, à l'exception des composés qui contiennent deux atomes d'oxygène et/ou de soufre voisins, l'hétérocycle pouvant encore être condensé sur un cycle benzénique ou sur un cycle hétéroaromatique à cinq ou six chaînons contenant un atome d'azote, d'oxygène ou de soufre, l'hétérocycle pouvant encore porter un atome d'halogène, un ou deux groupes alkyle en C1-C4 ou un groupe phényle ;
R² : l'hydrogène, un groupe cyano, un halogène, un groupe alkyle en C1-C4 ou alcoxy en C1-C4 ;
R³ : un groupe cyano ;
R⁴ et R⁵, indépendamment l'un de l'autre, l'hydrogène ou un groupe alkyle en C1-C4, l'un des deux symboles pouvant également représenter un groupe alcoxy en C1-C4 ;
Y : l'oxygène ou le soufre,
un groupe -SO-, -SO₂-, -CH₂-O-SO₂-, -N=N-, -O-CO-, - CO-O- ou -CO-O-CH₂,
une chaîne alkylène en C1-C4 qui peut être partiellement ou totalement halogénée et qui peut encore porter l'un des substituants suivants : cyano, nitro, alkyle en C1-C4, alkyle en C1-C4 partiellement ou totalement halogéné, alcényle en C2-C4, alcényle en C2-C4 partiellement ou totalement halogéné, alcoxy en C1-C4, phényle ou phénoxy, ces deux derniers groupes pouvant eux-mêmes porter un ou deux des substituants suivants : cyano, halogéno ou alkyle en C1-C4,
une chaîne alcénylène en C2-C4 ou alcynylène en C2-C4, une chaîne oxy-alkylène en C1-C4, thio-alkylène en C1-C4 ou alkylène-oxy en C1-C4 ou carbonyl-alkylène en C1-C4 ou alkylène en C2-C4-carbonyle.

4. Phénylacétamides substitués en ortho, répondant à la formule générale I.3 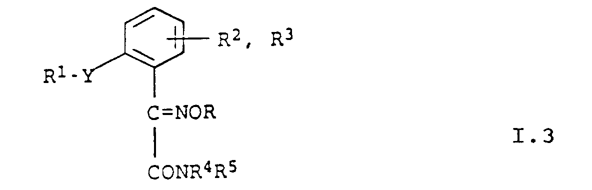 dans laquelle les symboles ont les significations suivantes :
R : un groupe alkyle en C1-C4,
R¹ : l'hydrogène, un groupe alkyle en C1-C18,
un groupe cycloalkyle en C3-C8 qui peut encore porter un à trois substituants choisis dans un groupe consistant en trois atomes d'halogènes, trois groupes alkyle en C1-C4, un groupe alcényle en C2-C4 partiellement ou totalement halogéné et un groupe phényle, lequel peut encore porter un à deux atomes d'halogènes et/ou un groupe alkyle en C1-C4 et/ou un groupe alcoxy en C1-C4,
un groupe alcényle en C2-C10,
un groupe alcynyle en C2-C4 qui peut porter un groupe phényle,
un groupe (alcoxy en C1-C4)alkyle en C1-C4, un groupe (alcoxy en C1-C4)carbonyle,
un groupe phényl-alkyle en C1-C4 ou phényl-alcényle en C2-C4 ou phénoxy-alkyle en C1-C4, chacune des parties aromatiques pouvant encore porter un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers groupes pouvant eux-mêmes porter un ou deux des substituants suivants : cyano, halogéno ou alkyle en C1-C4 :
le groupe phényle, qui peut encore porter un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers groupes pouvant eux-mêmes porter un ou deux des substituants suivants : cyano, halogéno ou alkyle en C1-C4 ;
un hétérocycle à cinq ou six chaînons contenant trois hétéroatomes choisis dans un groupe consistant en deux atomes d'oxygène, deux atomes de soufre et trois atomes d'azote, à l'exception des composés qui contiennent deux atomes d'oxygène et/ou de soufre voisins, l'hétérocycle pouvant encore être condensé sur un cycle benzénique ou sur un cycle hétéroaromatique à cinq ou six chaînons contenant un atome d'azote, d'oxygène ou de soufre, l'hétérocycle pouvant encore porter un atome d'halogène, un ou deux groupes alkyle en C1-C4 ou un groupe phényle ;
R², R³, indépendamment l'un de l'autre, l'hydrogène, un groupe cyano, un halogène, un groupe alkyle en C1-C4 ou alcoxy en C1-C4 ;
R⁴, R⁵, indépendamment l'un de l'autre, l'hydrogène ou un groupe alkyle en C1-C4, l'un des deux symboles pouvant encore représenter un groupe alcoxy en C1-C4 ;
Y : un groupe -SO₂-, -CH₂-O-SO₂-, -N=N-, -O-CO-, -CO-O- ou -CO-O-CH₂,
une chaîne alkylène en C1-C4 partiellement ou totalement halogénée ou qui peut en outre porter l'un des substituants suivants : cyano, nitro, alkyle en C1-C4, alkyle en C1-C4 partiellement ou totalement halogéné, alcényle en C2-C4, alcényle en C2-C4 partiellement ou totalement halogéné, alcoxy en C1-C4, phényle ou phénoxy, ces deux derniers groupes pouvant eux-mêmes porter un ou deux des substituants suivants : cyano, halogéno ou alkyle en C1-C4,
une chaîne carbonyl-alkylène en C1-C4 ou alkylène en C1-C4-carbonyle.

5. Phénylacétamides substitués en ortho, répondant à la formule générale I.4 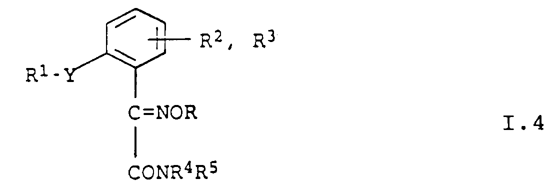 dans laquelle les symboles ont les significations suivantes :
R : un groupe alkyle en C1-C4,
R¹ : l'hydrogène, un groupe alkyle en C1-C18,
un groupe cycloalkyle en C3-C8 qui peut encore porter un à trois substituants choisis dans un groupe consistant en trois atomes d'halogènes, trois groupes alkyle en C1-C4, un groupe alcényle en C2-C4 partiellement ou totalement halogéné et un groupe phényle, lequel peut encore porter un à deux atomes d'halogènes et/ou un groupe alkyle en C1-C4 et/ou un groupe alcoxy en C1-C4,
un groupe (alcoxy en C1-C4)alkyle en C1-C4, un groupe (alcoxy en C1-C4)carbonyle,
R², R³, indépendamment l'un de l'autre, l'hydrogène, un groupe cyano, un halogène, un groupe alkyle en C1-C4 ou alcoxy en C1-C4 ;
R⁴, R⁵, indépendamment l'un de l'autre, l'hydrogène ou un groupe alkyle en C1-C4, l'un des deux symboles pouvant encore représenter un groupe alcoxy en C1-C4 ;
Y : l'oxygène ou le soufre,
un groupe -SO-, -SO₂-, -CH₂-O-SO₂-, -N=N-, -O-CO-, - CO-O- ou -CO-O-CH₂,
une chaîne alkylène en C1-C4 qui peut être partiellement ou totalement halogénée et qui peut encore porter l'un des substituants suivants : cyano, nitro, alkyle en C1-C4, alkyle en C1-C4 partiellement ou totalement halogéné, alcényle en C2-C4, alcényle en C2-C4 partiellement ou totalement halogéné, alcoxy en C1-C4, phényle ou phénoxy, ces deux derniers pouvant encore porter un ou deux des substituants suivants : cyano, halogéno ou alkyle en C1-C4,
une chaîne alcénylène en C2-C4 ou alcynylène en C2-C4, une chaîne oxy-alkylène en C1-C4, thio-alkylène en C1-C4 ou (alkylène en C1-C4)-oxy ou carbonyl-alkylène en C1-C4 ou (alkylène en C1-C4)carbonyle.

6. Phénylacétamides substitués en ortho, répondant à la formule générale I.5 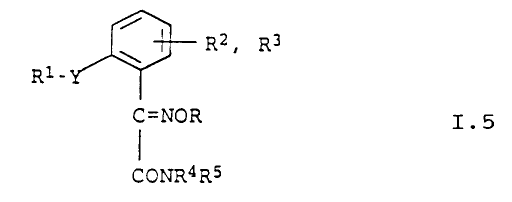 dans laquelle les symboles ont les significations suivantes :
R : un groupe alkyle en C1-C4,
R¹ : l'hydrogène, un groupe alkyle en C1-C18,
un groupe cycloalkyle en C3-C8 qui peint encore porter un à trois substituants choisie dans un groupe consistant en trois atomes d'halogènes, trois groupes alkyle en C1-C4, un groupe alcényle en C2-C4 partiellement ou totalement halogéné et un groupe phényle qui peut encore porter un à deux atomes d'halogènes et/ou un groupe alkyle en C1-C4 et/ou un groupe alcoxy en C1-C4,
un groupe alcynyle en C2-C4 qui peut porter un groupe phényle,
un groupe (alcoxy en C1-C4)alkyle en C1-C4, un groupe (alcoxy en C1-C4)carbonyle,
un groupe phényl-alkyle en C1-C4 ou phényl-alcényle en C2-C4 ou un groupe phénoxy-alkyle en C1-C4, la partie aromatique, dans chaque cas, pouvant encore porter un à cinq substituants choisis dans un groupe consistant en deux groupes nitro, deux groupes cyano, cinq atomes d'halogènes et trois de chacun des groupes suivants : alkyle en C1-C4, alkyle en C1-C4 partiellement ou totalement halogéné, alcényle en C2-C4, alcényle en C2-C4 partiellement ou totalement halogéné et alcoxy en C1-C4 et un groupe phényle, benzyle, phénoxy, benzyloxy ou phénylthio, ces deux derniers pouvant encore porter un ou deux des substituants suivants : cyano, halogéno ou alkyle en C1-C4 ;
un hétérocycle à cinq ou six chaînons choisi parmi les suivants : pyrrol-2-yle, pyrrol-3-yle, furan-2-yle, furan-3-yle, thién-2-yle, thién-3-yle, pyrazol-3-yle, pyrazol-4-yle, pyrazol-5-yle, oxazol-2-yle, oxazol-4-yle, oxazol-5-yle, benzoxazol-2-yle, thiazol-2-yle, thiazol-4-yle, thiazol-5-yle, benzothiazol-2-yle, isoxazol-3-yle, isoxazol-4-yle, isoxazol-5-yle, isothiazol-3-yle, isothiazol-4-yle, isothiazol-5-yle, isothiazol-3-yle, isothiazol-4-yle, isothiazol-5-yle, imidazol-2-yle, imidazol-4-yle, 1,2,4-oxadiazol-3-yle, 1,2,4-oxadiazol-5-yle, 1,2,4-thiadiazol-3-yle, 1,2,4-thiadiazol-5-yle, 1,3,4-oxadiazol-2-yle, 1,3,4-thiadiazol-2-yle, 1,2,4-triazol-3-yle, 1,3,4-triazol-2-yle, pyridin-3-yle, pyridin-4-yle, pyridazin-3-yle, pyridazin-4-yle, pyrimidin-4-yle, pyrimidin-5-yle, pyrazin-2-yle, 1,3,5-triazin-2-yle et 1,2,4-triazin-3-yle, l'hétérocycle pouvant encore porter un atome d'halogène, un ou deux groupes alkyle en C1-C4 ou un groupe phényle ;
R², R³, indépendamment l'un de l'autre, l'hydrogène, un groupe cyano, un halogène, un groupe alkyle en C1-C4 ou alcoxy en C1-C4 ;
R⁴, R⁵, indépendamment l'un de l'autre, l'hydrogène ou un groupe alkyle en C1-C4, l'un des deux symboles pouvant encore représenter un groupe alcoxy en C1-C4 ;
Y : l'oxygène ou le soufre, -SO-,
une chaîne alcénylène en C2-C4 ou alcynylène en C2-C4, une chaîne oxy-alkylène en C1-C4, thio-alkylène en C1-C4 ou (alkylène en C1-C4)-oxy.

7. Phénylocétamides substitués en ortho, répondant à la formule générale I.6 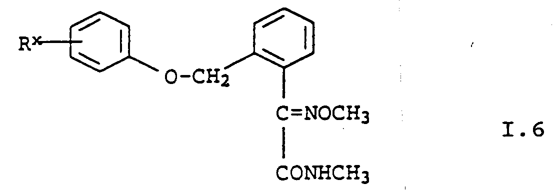 dans laquelle R^{x} représente les substituants et combinaisons de substituants ci-après :
2-fluoro, 3-fluoro, 2-bromo, 2-iodo, 2-méthyle, 3-méthyle, 2-méthoxy, 3-méthoxy, 2-trifluorométhyle, 4-trifluorométhyle, 2-nitron, 4-nitro, 2-chlorométhyle, 3-chlorométhyle, 4-chlorométhyle, 2-éthyle, 3-éthyle, 4-éthyle, 3-iso-propyle, 4-iso-propyle, 3-tert.-butyle, 3-phényle, 4-phényle, 3,5-diéthyle, 4-cyclohexyle, 4-isopropyloxy, 4-tert.-butyloxy, 4-phénoxy, 3-benzyloxy, 4-benzyloxy, 2,3-dichloro, 2,5-dichloro, 2,6-dichloro, 2,3,4-trichloro, 2,3,5-trichloro, 2,3,6-trichloro, 3,4,5-trichloro, pentachloro, pentafluoro, 2-fluoro-4-chloro, 4-fluoro-2-chloro, 2-méthyl-4-tert.-butyle, 2-méthyl-4-cyclohexyle, 2-méthyl-4-propyle, 2,3-diméthyle, 2,4-diméthyle, 2,5-diméthyle, 3,4-diméthyle, 3,5-diméthyle, 2,3,5-triméthyle ou 2,4,6-triméthyle.

8. Phénylacétamides substitués en ortho, répondant à la formule générale I.7 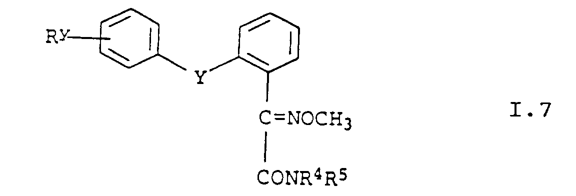 dans laquelle les symboles ont les significations suivantes :
R⁴, R⁵, indépendamment l'un de l'autre, l'hydrogène ou un groupe méthyle ;
Y : CH₂CH₂, CH=CH ou CH₂O,
R^{y} : 2-fluoro, 3-fluoro, 4-fluoro, 2-chloro, 3-chloro, 4-chloro, 2-bromo, 4-bromo, 2-jodo, 2-méthyle, 3-méthyle, 4-méthyle, 2-méthoxy, 3-méthoxy, 4-méthoxy, 2-trifluorométhyle, 3-trifluorométhyle, 4-trifluorométhyle, 2,4-dichloro, 2,4-diméthyle ou 2,4,6-triméthyle.

9. Procédé de préparation des composés I.1 à I.7 selon les revendications 2 à 8, caractérisé par le fait que l'on fait réagir un dérivé de l'acide phénylacétique répondant à la formule II 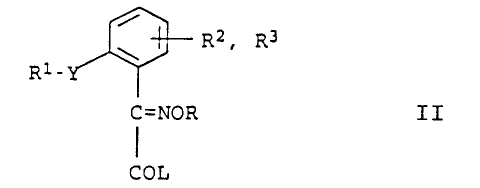 dans laquelle L représente un halogène ou un groupe alcoxy en C1-C4, le cas échéant en présence d'une base, avec une amine de formule III
HNR⁴R⁵ III

10. Procédé de préparation des composés I.1 à I.7 selon les revendications 2 à 8, caractérisé par le fait que l'on hydrolyse un phénylacétonitrile de formule IV 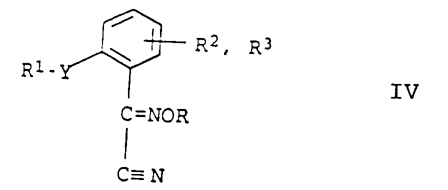 en présence d'un acide ou d'une base, et on soumet le produit obtenu à une monoalkylation sur l'atome d'azote d'amide.

11. Phénylacétonitriles de formule IV selon la revendication 10.

12. Utilisation des composés IV selon la revendication 11 en tant que produits intermédiaires.

13. Produit fongicide contenant un véhicule solide ou liquide et au moins un composé de formule I.1 à I.7 selon les revendications 2 à 8.

14. Produit parasiticide contenant un véhicule inerte et au moins un composé de formule I.1 à I.7 selon les revendications 2 à 8.

15. Procédé pour combattre les mycètes, caractérisé par le fait que l'on fait agir une quantité fongicide efficace d'un composé de formule I.1 à I.7 selon les revendications 2 à 8 sur les mycètes ou sur les végétaux, leur habitat ou les semences des végétaux menacés d'une attaque par les mycètes.
